# EUROPEAN PATENT APPLICATION

(11) **EP 1 655 306 A1**
(43) Date of publication of application: **10.05.2006**
(21) Application number: 05256580.1
(22) Date of filing: 24.10.2005
(51) Int. Cl.: C07K 14/435, A61K 39/38, A61K 48/00

(54) **Protective antigenes and vaccines for the control of multi species tick infestations**

(30) Priority: 25.10.2004 US 972789
(71) Applicant: THE BOARD OF REGENTS OF OKLAHOMA STATE UNIVERSITY, Stillwater, OK 74078 (US)
(72) Inventor: De la Fuente, Jose de Jesus, Stillwater, OK 74074 (US); Kocan, Katherine M., Perkins, OK 74059 (US); Garcia-Almazan, Consuelo, Tam. CP 87037 (MX); Blouin, F. Edmour, Perkins, OK 74059 (US)
(74) Representative: Williams, Aylsa

(57) **Abstract**

Broad range protective antigens against tick infestations, gene sequences and encoded proteins for such antigens, related vaccines and methods useful to induce an immune response.

SEQUENCE LISTING

<110>  De La Fuente, Jose de Jesus
       Kocan, Katherine M.
       Garcia-Almazan, Consuelo
       Blouin, Edwin F.

<120>  Protective Antigens and Vaccines for the Control of Multi Species
       Tick Infestations

<130>  04-597

<150>  10/425,563
<151>  2003-04-29

<150>  60/376,251
<151>  2002-04-29

<160>  39

<170>  PatentIn version 3.2

<210>  1
<211>  349
<212>  DNA
<213>  Ixodes scapularis

<400>  1
atggaaatat ctgtgaaacc aaggcccaca aaaaggaaaa gaaaggccat catcatcatg      60

gcaagaatga gaacagcatt ccccaccaga agtgggaaca gcttctcaag gacttgaaca     120

gttaatgatg tgttgtgcaa ttcgaatgtg gctgcaacct cgctagagaa catagtcgac     180

cagctgtagt gctctagtat taaccaagaa gcagtattct gccgtcatat gtacaggcag     240

atttgttacg gcattttcag ctttttttta tacaaaatgt agttcttgtt taaaaaaaac     300

ctaaaataaa acaaagccac aaaaaaaaaa aaaaaaaaaa aaaaaaaaa                349

<210>  2
<211>  38
<212>  PRT
<213>  Ixodes scapularis

<400>  2

Met Glu Ile Ser Val Lys Pro Arg Pro Thr Lys Arg Lys Arg Lys Ala
1               5                   10                  15

Ile Ile Ile Met Ala Arg Met Arg Thr Ala Phe Pro Thr Arg Ser Gly
            20                  25                  30

Asn Ser Phe Ser Arg Thr
        35

<210>  3
<211>  2693
<212>  DNA
<213>  Ixodes scapularis

<220>

<221>  misc_feature
<222>  (685)..(685)
<223>  n is a, c, g, or t

<220>
<221>  misc_feature
<222>  (1962)..(1962)
<223>  n is a, c, g, or t

<400>  3
ggtttgtgct gcttggaaaa tccaccagga gcctgcaacc gcaaaaaagt tcatcatccg      60

gagctaagcc tatcgcagga tggcttgcgc aacattaaag cgaacacacg attgggatcc     120

gctgcatagt ccaaacggaa gatcgcccaa acgacggaga tgtatgcctt tgtcggtcac     180

acaagcagcg actcccccaa caagggcaca ccaaatcaac ccttcaccct tcggtgaagt     240

gccaccgaaa ttaacttcag aggagatagc ggccaacatt cgggaggaaa tgcgacgtct     300

gcagcggcgc aagcagctct gcttctcgtc tcccctggag tcgggctccc cgtcggcgac     360

tccccctgcg gccgattgcg gaccagcctc ccccacgggc ctgtcccccg ggggcctgct     420

gtcgcccgtg cgcagggacc aacccctctt caccttccgc caggtggggc tcatctgcga     480

gcgcatgatg aaggagcgcg agagccagat acgcgacgag tacgaccacg ttctgtctgc     540

caagctggca gagcagtacg acacatttgt caagtttacg tacgaccaaa ttcagaagcg     600

gtttgagggt gccactccaa gctatttgtc ataacatgat gggcatctgc aaacaagcaa     660

ggaactttga gggtttgtgc taganggaag aaacccatgg tggggaagga cacaagacca     720

acacttagac tcggcaagca agccagatcc tgtggggtgc ggggacgggg ggaatgagtc     780

cagtggtgtc ttcggagttt ttttttttcc ttctcccttt ccctcgtctt ctttttggca     840

caactcttta cggaactggt gtgcatccat tccccgaaag tgcaagagaa ggactcgcgg     900

cggatcatct acggaggaag aagtgtgtat gcctttgtgc tttgggtctc cttttttttt     960

tttttaaccg tcttgccatc tcgccataga agacctgtga tctagcaaac aaaggtgtgc    1020

gaatgttatg caaaggttgg aagtcagttt gaaagtggag cgagagaaaa ttttgtatgc    1080

tgagtatggt tagtcaccgt ttacttttca ggagggggat gactgaggaa cggagccgcc    1140

ccaactctcg tttgtctttt atttttagga tactttctct gtggcgagaa tttgtgtgtg    1200

catgcaagtt agcgagggta cgaggaaaag aagggttata aaatattctg ggtgagagct    1260

gtagttcaac tggggggtgg gattgtaaag acctgcgggt accgagagga ccgcatgctc    1320

tggctatatt acttgcattg agggggagga ggaatgctgg acctcgagca gagccagcaa    1380

gtattttgga aaggaataaa aacaaaaatt ggcttagtgt acagatgtat aatatatatg    1440

cactacaggg tgtgtgtgcc tcttgtatct tcccgtgcgt tgtgtccctc ttgcggcttg    1500

cccatctgac aaccgcctgt gtacataggc caacgcaagt cttcagcatg gcaccctctt    1560

ctttttcctt ttttttttct cataagtaat tttgaaggag agaatatttt gatttctaag    1620

actcccaaaa catcaagtgc tctggtggtc ggaattctac aagtgcgaaa gttcctttct    1680

tttttttgtt tcgagatagg aatggcttca ggttgtgctg cctatgcttt ggccacactt    1740

tggaacacct gcaacagcga attaactggt gtaggcctgt gacacttgca cagccgtgtt 1800
tttttttttt ttttttgtag ttttgcagta ataaaaactt gttatggaaa gagtgcatta 1860
tgctatggca ttgtctgctg ctatgcttat tggaatgcat gcctgatgtg tgttgtgctt 1920
gaggatagtg aagtggtatt gcagggttgg aaaggagctt anaatgcctt ctggcttttg 1980
cataagcgtg gctttgggtg tcgtctgagc ttgtcaatca cagtgcaaca tgcactttgt 2040
ccaattggtt tattggggac tgcttttggg tgcagagttt gactaatttt tagtaatgct 2100
tcaaatgcaa cgcttctgtg ttgatcgcag ttcatcaact cgtcgatcat tatgcatgtg 2160
aaaaactgct cacgtaaact gtatgttgat atcacagttg cactgaggaa gcctggctta 2220
agatggtgtg tgcaagtgct tggcacactg cgtattttcc agcataaagc tggtagtgta 2280
caggtgctgc tgttagtagc aaactttctg ccattgctgc cacaattcat gcatgaatga 2340
gtgttgggga gtatgttagt ttatcttttc aaaactgatt tgaagtacca gtgtcctata 2400
tttttgccat tgcattaata tggatcctgc attgtttacg gaaaaaagtg ataacattaa 2460
ttatgaaaga tattaagcga gtttattgac ttttccagga gaatttagac caggaggcac 2520
tacatagcct gtggtctgct ttgttatgtt gacttggttt ttgtggaaat tagttctaaa 2580
agtttacaat ctttttggca tgacttgttt gcattgccat tgtaatttgg ccattattag 2640
aataaaggca ctctctcagt acctaaaaaa aaaaaaaaaa aaaaaaaaaa aaa 2693

<210> 4
<211> 184
<212> PRT
<213> Ixodes scapularis

<400> 4

Met Ala Cys Ala Thr Leu Lys Arg Thr His Asp Trp Asp Pro Leu His
1               5                   10                  15

Ser Pro Asn Gly Arg Ser Pro Lys Arg Arg Arg Cys Met Pro Leu Ser
            20                  25                  30

Val Thr Gln Ala Ala Thr Pro Pro Thr Arg Ala His Gln Ile Asn Pro
        35                  40                  45

Ser Pro Phe Gly Glu Val Pro Pro Lys Leu Thr Ser Glu Glu Ile Ala
    50                  55                  60

Ala Asn Ile Arg Glu Glu Met Arg Arg Leu Gln Arg Arg Lys Gln Leu
65                  70                  75                  80

Cys Phe Ser Ser Pro Leu Glu Ser Gly Ser Pro Ser Ala Thr Pro Pro
                85                  90                  95

Ala Ala Asp Cys Gly Pro Ala Ser Pro Thr Gly Leu Ser Pro Gly Gly
            100                 105                 110

Leu Leu Ser Pro Val Arg Arg Asp Gln Pro Leu Phe Thr Phe Arg Gln
        115                 120                 125

Val Gly Leu Ile Cys Glu Arg Met Met Lys Glu Arg Glu Ser Gln Ile
    130                 135                 140

Arg Asp Glu Tyr Asp His Val Leu Ser Ala Lys Leu Ala Glu Gln Tyr
145                 150                 155                 160

Asp Thr Phe Val Lys Phe Thr Tyr Asp Gln Ile Gln Lys Arg Phe Glu
                165                 170                 175

Gly Ala Thr Pro Ser Tyr Leu Ser
            180

<210> 5
<211> 1821
<212> DNA
<213> Ixodes scapularis

<220>
<221> misc_feature
<222> (1487)..(1487)
<223> n is a, c, g, or t

<220>
<221> misc_feature
<222> (1595)..(1595)
<223> n is a, c, g, or t

<220>
<221> misc_feature
<222> (1606)..(1606)
<223> n is a, c, g, or t

<220>
<221> misc_feature
<222> (1623)..(1623)
<223> n is a, c, g, or t

<220>
<221> misc_feature
<222> (1762)..(1762)
<223> n is a, c, g, or t

<220>
<221> misc_feature
<222> (1789)..(1789)
<223> n is a, c, g, or t

<400> 5
atggcgtcgt gtggagcatc agcgacgggt cctctcgtcc taagagtaat ttccaacact 60

gttaaaatag ttaacagcgc cggaaagata atcaaggaca tcatgaacag tggaaacctc 120

ggaatcgtcg aaaaggaagg catcaatgac ctgcaaacgg aggcagacag atctgttcag 180

cgctgcattg tgacttcgct ctcgagacag ttcccaaaac tgacaataat tggtgaagag 240

actctggagg agaaaaagat cagcgacgac tggatcatca ccgagcatga caaggatgtc 300

ctggccactt ctctgccgga caacctgaag aacatcaaag aggaagattt ggtagtctgg 360

gttgatcctc tggatggaac caaggagtac acacagggtt tcctggacca cgtgacgatc 420
ctggtgggga ttgcggttga cggtaaggca gtgggtggag tgatccacca gccgtactac 480
aactaccagg tggagaagga cgtctacaag cagggacgta ccatgtgggg cattgtcggc 540
gtcggtgcct ttgggatctc gcgcattgcg cctccggaga acaagaggat catcactacg 600
acgcgctccc attccagccc caccatcaac agctgcattg aagccatgaa tccggacgag 660
gtgctgcgag ttggaggtgc cgggcacaag gtgctgctgt tgattgaggg caaggctcac 720
gcttacgtgt ttcccagcaa agggtgcaag aaatggacca cttgtgcccc cgaagcgatt 780
cttcatgcca ctggcggcct tcttacagat gttcacggga acagattgga gtaccacaag 840
gacgtggaac acgtcaatgc cggcggcgtt cttgccacct gcctgaagga acaacacgaa 900
tggttcaaga accacattcc cgaagatgtc cgcaagacgc ttcctctatg agcaacctgc 960
cgttgtccgt tgcgatcaca ctcaagtcgc gtttttcctt taactttgtg gtgatgcggt 1020
tcaaagtctt atactattag tgttttggtg gtccaaatat tattactaaa aaaacccgga 1080
gacatgggac acaaaaaaat ggaggggcgg gacaataagg tctcgaacac agctcgtaca 1140
gaatttttta aaataatgtt gatttcaggt ttatttgtgg aaactctgaa attaaccgtt 1200
atgtcattat ttgggttgtg ccgtttgaaa ttttatgaaa tacgtaatag ctgcacgcat 1260
tttgcaggcc actcagctcc ttgaatgctc gatgcttgat gcttctgcca acattatttg 1320
tatctcaagt ttttctacca caagaaacag taccctaaca ttttgaaata gtattactag 1380
cttggatttt atctggtatg catatataag atctatggat gttcctaagg agggcatgaa 1440
tttgaaacat accctgtcct taccaacctt caaacatttt tttttgngcc tgcttaaaag 1500
cacttacatt gcttgatcgt tgaattaatt ttttagctga tgttaaggac acttataata 1560
attaaggaaa tgagatcgat cttgagcttg tttgngcctc tgtaanaatt gatgctcttt 1620
canacctaat gcttaatgca acaataatta tcaagtaatc cttactcagg tgtcagatat 1680
gcaagcagat gccaatgctt ctgttcattg agtggcaaaa ggcattgctc tttgtcacat 1740
tgcatgcatt tatgacagcc cnccttaata aactataatg cagctaatnt gaaaaaaaaa 1800
aaaaaaaaaa aaaaaaaaaa a 1821

<210> 6
<211> 316
<212> PRT
<213> Ixodes scapularis

<400> 6

Met Ala Ser Cys Gly Ala Ser Ala Thr Gly Pro Leu Val Leu Arg Val
1               5                   10                  15

Ile Ser Asn Thr Val Lys Ile Val Asn Ser Ala Gly Lys Ile Ile Lys
            20                  25                  30

Asp Ile Met Asn Ser Gly Asn Leu Gly Ile Val Glu Lys Glu Gly Ile

35                  40                  45

Asn Asp Leu Gln Thr Glu Ala Asp Arg Ser Val Gln Arg Cys Ile Val
    50                  55                  60

Thr Ser Leu Ser Arg Gln Phe Pro Lys Leu Thr Ile Ile Gly Glu Glu
65                  70                  75                  80

Thr Leu Glu Glu Lys Lys Ile Ser Asp Asp Trp Ile Ile Thr Glu His
                85                  90                  95

Asp Lys Asp Val Leu Ala Thr Ser Leu Pro Asp Asn Leu Lys Asn Ile
            100                 105                 110

Lys Glu Glu Asp Leu Val Val Trp Val Asp Pro Leu Asp Gly Thr Lys
        115                 120                 125

Glu Tyr Thr Gln Gly Phe Leu Asp His Val Thr Ile Leu Val Gly Ile
    130                 135                 140

Ala Val Asp Gly Lys Ala Val Gly Gly Val Ile His Gln Pro Tyr Tyr
145                 150                 155                 160

Asn Tyr Gln Val Glu Lys Asp Val Tyr Lys Gln Gly Arg Thr Met Trp
                165                 170                 175

Gly Ile Val Gly Val Gly Ala Phe Gly Ile Ser Arg Ile Ala Pro Pro
            180                 185                 190

Glu Asn Lys Arg Ile Ile Thr Thr Thr Arg Ser His Ser Ser Pro Thr
        195                 200                 205

Ile Asn Ser Cys Ile Glu Ala Met Asn Pro Asp Glu Val Leu Arg Val
    210                 215                 220

Gly Gly Ala Gly His Lys Val Leu Leu Leu Ile Glu Gly Lys Ala His
225                 230                 235                 240

Ala Tyr Val Phe Pro Ser Lys Gly Cys Lys Lys Trp Asp Thr Cys Ala
                245                 250                 255

Pro Glu Ala Ile Leu His Ala Thr Gly Gly Leu Leu Thr Asp Val His
            260                 265                 270

Gly Asn Arg Leu Glu Tyr His Lys Asp Val Glu His Val Asn Ala Gly
        275                 280                 285

Gly Val Leu Ala Thr Cys Leu Lys Glu Gln His Glu Trp Phe Lys Asn
    290                 295                 300

His Ile Pro Glu Asp Val Arg Lys Thr Leu Pro Leu

305                 310                 315

<210>  7
<211>  697
<212>  DNA
<213>  Ixodes scapularis

<220>
<221>  misc_feature
<222>  (573)..(573)
<223>  n is a, c, g, or t

<400>  7
gacctcaagg aaggcatctg cccgcaggcc ttctggctca acaaggagca gtgttgctgg      60

gcctccaacg ataccttctt taagggggac gactgcaagc agtggtatcg gtggcccgag     120

atgttcgaca gcggcatgga caaggacggg gcaggctttt acctgctctc ctacctgctg     180

tacgtcatgt ggagtgtgct cttcgccacc ctggccgtca tgctcgttcg caccttcgcg     240

ccctatgcct gtggatctgg aatcccggag atcaagacga ttctgagcgg cttcatcatc     300

cgcggctacc tgggcaagtg gacgctgacc atcaaatcag tgtgtctggt gctggccgtc     360

ggggcgggcc tcagcctggg caaagagggg cccctggtgc acgtggcctg ctgcatcggg     420

aacatcttct cctacctctt ccccaagtac ggcaagaatg aggccaagaa gagggagatc     480

ctgtcggctg ccgccgccgc gggagtttct gtggcctttg gggctcccat cggcggtgtt     540

ctcttcagcc tcgaagaggt gagctactac ttncccttga agacgctgtg gcgttccttc     600

ttctgcgccc tggtggcagc ctcggtgctg cgctccatca acccctttgg caacgaccac     660

ctggtgatgt tctacgtcga gtacgacttt ccctggc                              697

<210>  8
<211>  232
<212>  PRT
<213>  Ixodes scapularis

<220>
<221>  misc_feature
<222>  (191)..(191)
<223>  Xaa can be any naturally occurring amino acid

<400>  8

Asp Leu Lys Glu Gly Ile Cys Pro Gln Ala Phe Trp Leu Asn Lys Glu
1               5                   10                  15

Gln Cys Cys Trp Ala Ser Asn Asp Thr Phe Phe Lys Gly Asp Asp Cys
            20                  25                  30

Lys Gln Trp Tyr Arg Trp Pro Glu Met Phe Asp Ser Gly Met Asp Lys
        35                  40                  45

Asp Gly Ala Gly Phe Tyr Leu Leu Ser Tyr Leu Leu Tyr Val Met Trp
    50                  55                  60

Ser Val Leu Phe Ala Thr Leu Ala Val Met Leu Val Arg Thr Phe Ala
65                  70                  75                  80

Pro Tyr Ala Cys Gly Ser Gly Ile Pro Glu Ile Lys Thr Ile Leu Ser
                85                  90                  95

Gly Phe Ile Ile Arg Gly Tyr Leu Gly Lys Trp Thr Leu Thr Ile Lys
            100                 105                 110

Ser Val Cys Leu Val Leu Ala Val Gly Ala Gly Leu Ser Leu Gly Lys
        115                 120                 125

Glu Gly Pro Leu Val His Val Ala Cys Cys Ile Gly Asn Ile Phe Ser
    130                 135                 140

Tyr Leu Phe Pro Lys Tyr Gly Lys Asn Glu Ala Lys Lys Arg Glu Ile
145                 150                 155                 160

Leu Ser Ala Ala Ala Ala Ala Gly Val Ser Val Ala Phe Gly Ala Pro
                165                 170                 175

Ile Gly Gly Val Leu Phe Ser Leu Glu Glu Val Ser Tyr Tyr Xaa Pro
            180                 185                 190

Leu Lys Thr Leu Trp Arg Ser Phe Phe Cys Ala Leu Val Ala Ala Ser
        195                 200                 205

Val Leu Arg Ser Ile Asn Pro Phe Gly Asn Asp His Leu Val Met Phe
    210                 215                 220

Tyr Val Glu Tyr Asp Phe Pro Trp
225                 230

<210>  9
<211>  1221
<212>  DNA
<213>  Ixodes scapularis

<220>
<221>  misc_feature
<222>  (713)..(713)
<223>  n is a, c, g, or t

<400>  9
gtttcttgtt acggtagtgg agtgctgagt ttactcgata atatctgaga aatagtggca      60

atatcaattt ttctgtaaat tagaaatgta accaatggcg tggctatctt ctagtcgaca     120

ctaacgtctc ggatctgctg ttcaaagtcc cgggcgatca agccgtattt gttgtccagc     180

tgccaagtgc gtcgaatatg atgccgaaaa agaaagaatc agtcgcgagc tctaaagaag     240

acgcgccgat cgacgtgatc ggcctgccct cccacaaacg acacaagaag cacaagcaca     300

aaaagcacaa gcgcaagcga ggcacggacc aagacgaaga ccaatcgccc gccgcgagcc     360

cgcagagcgg tggcgagggt agcagcagca agcccgcgct caagctcaag atcaagatcg 420
gcggacagac ggtcgagaag aacgtgacca agctgaaaca gcagcggccg ccgccgccgg 480
accctagcga agccgatctc gccgaactcc tgatgaaacc caactcgggc gatacgagcg 540
cagacagcga tgacgaagag gaagcctggc tcgaagccct cgagtccggc aggctcgaag 600
aggtcgacga cgagctccgc aaaatgaagg acccgaccct gatgacggcc aggcagcggg 660
ccctgctcga gagcaagtcg cagaaggacg aggtcccggc gacggggatg gcnggcgtcc 720
gcggagcccg tcaaagagat gtccgaggag atgattcagc ggcggatgct gcgggccaaa 780
aagcggaagc agcaggccga agagaagaaa gagaaggaga agaagcagac gatcgagcgt 840
ctgctcaaga agtccgactc gaggctgagg gccagcaaga agttggccaa gaagagcgat 900
actcccaagg tgtcgctggt caacacgcag gcaggcacgc tgctctcgtt tcccgtcggc 960
gttgcgttcc cgctgtcggc agccgtggcc caggggtacc ccgagaagac gacgtgcggc 1020
attaagggtt gtcgtaaccc gaagaagtac tcgtgctcca agacaggcgt gcccctgtgc 1080
agcctcgagt gctacaagac gaacatgctg cagatgtgcg tctgagcggg cagctaggct 1140
tccgggctac agctgctcct tgtgtatatg tatataaagt cgagaatgct gaaaaaaaaa 1200
aaaaaaaaaa aaaaaaaaaa a 1221

<210> 10
<211> 275
<212> PRT
<213> Ixodes scapularis

<400> 10

Met Met Pro Lys Lys Lys Glu Ser Val Ala Ser Ser Lys Glu Asp Ala
1               5                   10                  15

Pro Ile Asp Val Ile Gly Leu Pro Ser His Lys Arg His Lys Lys His
            20                  25                  30

Lys His Lys Lys His Lys Arg Lys Arg Gly Thr Asp Gln Asp Glu Asp
        35                  40                  45

Gln Ser Pro Ala Ala Ser Pro Gln Ser Gly Gly Glu Gly Ser Ser Ser
    50                  55                  60

Lys Pro Ala Leu Lys Leu Lys Ile Lys Ile Gly Gly Gln Thr Val Glu
65                  70                  75                  80

Lys Asn Val Thr Lys Leu Lys Gln Gln Arg Pro Pro Pro Pro Asp Pro
                85                  90                  95

Ser Glu Ala Asp Leu Ala Glu Leu Leu Met Lys Pro Asn Ser Gly Asp
            100                 105                 110

Thr Ser Ala Asp Ser Asp Asp Glu Glu Glu Ala Trp Leu Glu Ala Leu

115                 120                 125

Glu Ser Gly Arg Leu Glu Glu Val Asp Asp Glu Leu Arg Lys Met Lys
    130                 135                 140

Asp Pro Thr Leu Met Thr Ala Arg Gln Arg Ala Leu Leu Glu Ser Lys
145                 150                 155                 160

Ser Gln Lys Asp Glu Val Pro Ala Thr Gly Met Ala Gly Val Arg Gly
                165                 170                 175

Ala Arg Gln Arg Asp Val Arg Gly Asp Asp Ser Ala Ala Asp Ala Ala
            180                 185                 190

Gly Gln Lys Ala Glu Ala Ala Gly Arg Arg Glu Glu Arg Glu Gly Glu
        195                 200                 205

Glu Ala Asp Asp Arg Ala Ser Ala Gln Glu Val Arg Leu Glu Ala Glu
    210                 215                 220

Gly Gln Gln Glu Val Gly Gln Glu Glu Arg Tyr Ser Gln Gly Val Ala
225                 230                 235                 240

Gly Gln His Ala Gly Arg His Ala Ala Leu Val Ser Arg Arg Arg Cys
                245                 250                 255

Val Pro Ala Val Gly Ser Arg Gly Pro Gly Val Pro Arg Glu Asp Asp
            260                 265                 270

Val Arg His
        275

<210>  11
<211>  1942
<212>  DNA
<213>  Ixodes scapularis

<400>  11
cgatgcaggc gatgacgggc tttgcggtgc agttcaacaa aaacagtttc gggctgactc      60

cagctcagcc gctgcagttg cagattcccc tgcagcccaa cttcccagct gatgcgagct     120

tgcagctggg aaccaacggt cccgtgcaga agatggaccc cctcaccaac cttcaggtgg     180

ccatcaagaa caatgtggac gtgttctact tcagctgcct ggtgcccatg cacgtgctga     240

gcacggagga cggcctgatg gacaagcggg tgttcctggc cacctggaaa gacatccccg     300

cccaaaacga ggtccagtac accctcgaca acgtcaacct cactgcagac caagtttccc     360

agaagctgca gaacaacaac attttcacga tagccaagag gaacgtggac ggccaggaca     420

tgctgtacca gtccctgaag ctcaccaacg gcatttgggt gttggcggag ctcaagatac     480

agcccggcaa tccaaggatc acgttgtctt tgaagacaag agcacctgaa gtggcagcag     540

gtgtacaaca aacttacgaa ctcattctac acagctgagg ctgctgtgaa tgaaactctt     600

ctcccacccc cttcttttga tggcagtcaa tgtctcgttt cattttcttg ttttcttttg 660

cggcgtgcta cggaacaagg tcctacattc ccaagttata tggtgttgtc gcgtaggggg 720

cagagtgccg ctgagcccgc gacagccttg tttctgagga gagccgaacg caccacttcg 780

aaaaagaaaa agtgaaaacg gaaaaatgaa aaattttcca gttgcttcaa attaacattc 840

ctcgtagtca gtctgtggcc gttgagtttg gtgtaaagaa gaaaaaggtg tctcttttag 900

tgaaaatggt tgctttttat tggtatcccc tatcacaccg agcacgaaca taagaaatcc 960

tgacaaggat tctcctttag ttgtattatg gtggctggag cacacgaggc acctgttgcc 1020

aattcgaccc agcaaatgcc caattctcaa gatttgagtt cattgaggtt gttttgctcc 1080

tcccccccca ccccccaact ttgtcgttgg attgtctaac agtgtaaatg ggcgacgact 1140

cgttattctt tttttcttca ttctttcttt ttgttgtcac gcgccccggg ggacgcgaca 1200

caacttatgt gcataattga ttttcacagg ctgcgacgca gtctgtaaaa gaaggggaag 1260

tgaaactctg ctccgccgct gctagtgtca tcacgggacg accatcgcgt tttctctgac 1320

tatttaaaca aaactgcata gcttaggggg cagtctgtgc aaagtggaac aaccaaactg 1380

agccctgccc tttcggtgtg tgtacaagca tctctgtgta acatgaacta ctttacatga 1440

actacattgc atgaacggga gaagtttagt tgtttttttg tttttttttt caggtgacta 1500

tgtcaacaga ttagaaccat tttttggaac ggctggaaag ataaccgctc attttgtttc 1560

tactaaaaga ctacgaaaag tgttgacttt ttgcatcggt ttggcaacgt ttgtttggca 1620

tgcatgtagt tgagcgtaat ggtatcaccc ctcgtaaaca ataacagtgc aatggagcag 1680

tactgtagtg tccattaaag agcgagagtt tggttaaagg ttgttaattg aggtccgtgt 1740

tatcctttga gtaggagagc ggcacttttt gcaaatagcg ctgctggggg cgtcatatct 1800

gccctccaaa acatgcacat tttaagtgtg aattgttgcg gcggcttgta caagtatgtg 1860

tgttatgtgt agaaaaagaa ctcttaatta aaatatttgt ggccaaaacg tcaaaaaaaa 1920

aaaaaaaaaa aaaaaaaaaa aa 1942

<210> 12
<211> 191
<212> PRT
<213> Ixodes scapularis

<400> 12

Met Gln Ala Met Thr Gly Phe Ala Val Gln Phe Asn Lys Asn Ser Phe
1               5                   10                  15

Gly Leu Thr Pro Ala Gln Pro Leu Gln Leu Gln Ile Pro Leu Gln Pro
            20                  25                  30

Asn Phe Pro Ala Asp Ala Ser Leu Gln Leu Gly Thr Asn Gly Pro Val
        35                  40                  45

Gln Lys Met Asp Pro Leu Thr Asn Leu Gln Val Ala Ile Lys Asn Asn

50                  55                  60

Val Asp Val Phe Tyr Phe Ser Cys Leu Val Pro Met His Val Leu Ser
65                  70                  75                  80

Thr Glu Asp Gly Leu Met Asp Lys Arg Val Phe Leu Ala Thr Trp Lys
                85                  90                  95

Asp Ile Pro Ala Gln Asn Glu Val Gln Tyr Thr Leu Asp Asn Val Asn
            100                 105                 110

Leu Thr Ala Asp Gln Val Ser Gln Lys Leu Gln Asn Asn Asn Ile Phe
        115                 120                 125

Thr Ile Ala Lys Arg Asn Val Asp Gly Gln Asp Met Leu Tyr Gln Ser
    130                 135                 140

Leu Lys Leu Thr Asn Gly Ile Trp Val Leu Ala Glu Leu Lys Ile Gln
145                 150                 155                 160

Pro Gly Asn Pro Arg Ile Thr Leu Ser Leu Lys Thr Arg Ala Pro Glu
                165                 170                 175

Val Ala Ala Gly Val Gln Gln Thr Tyr Glu Leu Ile Leu His Ser
            180                 185                 190

<210>  13
<211>  1428
<212>  DNA
<213>  Ixodes scapularis

<220>
<221>  misc_feature
<222>  (701)..(701)
<223>  n is a, c, g, or t

<400>  13
   cgcgccgtgc agaagctgcg tcgggaggtt gagaaggcaa agaggaccct gtccactgct      60
   caccaggcca ggatcgagat tgaatcgttc ttcgagggag aggacttcag tgagaccctg     120
   actcgtgcta agtttgagga gctgaacatg gaccttttcc gttccaccat gaagcctgtt     180
   cagaaggtac tcgaggatgg tgacctcaag aagactgatg tggacgagat tgtgcttgtc     240
   ggaggttcca ccaggatccc caaggttcaa cagctggtca aggagttctt caatggcaag     300
   gaacccaccc gtggcatcaa ccccgacgaa gcagtcgcct acggtgccgc cgtgcaggct     360
   ggagtcctcg gcggagagga agacactggg gacctcgtgc tgttggacgt gaaccctctg     420
   accctcggca tcgagacagt gggaggcgtc atgacgaaac tgatcccccg taacacagtc     480
   atccccacga agaagtctca gatcttctcc acggcctcgg acgagcagag cactgtcacc     540
   atccaggtct ttgaggggga gcgtcccctg acaaaggaca accaccagct gggcaagttc     600
   gacctgactg gcatcccacc tgctcctcga ggtgtgcccc aaatcgaggt gaccttcgag     660
   attgacgtca acggtatcct gcgggtcagt gcagaggaca ngggtacagg caacaagcag 720
   aagatcacca tcaacaatga ccagaacagg ctgacgcctg aggacatcga gaggatggta 780
   aaggacgccg aaaagtttgc cgacgaggac aagaaggtca aggagaaggt ggaggcccgc 840
   aacgaactgg agtcttatgc ctactccctc aagaaccaga ttggagacaa ggagaagatg 900
   ggaggcaagc tctccgacga ggacaagaag actattgagc aagctgtgga cgagaaaatc 960
   aaatggctgg agcagcacag tgacgctgat gcggaagaac tcaaggaaca gaagaaacag 1020
   ctggctgata ctgtgcagcc gattgtagcc aagctgtacc ctgcaggagg caccccaccg 1080
   ccgacggaca aagatgactc tacaaaggac gagttgtaaa aacaaggcca gatctcttgg 1140
   gtacagcgaa aggcatgggg cagcagcatt atcacaagtc atctgttacg atcatgagct 1200
   catcatttca ccacctctac agtgctgctg ctgcctgcct tttggctggt tgagtgttct 1260
   tggacctatt taccatgatc attctctgta caaaaacaat tctttctgtg tttttttttt 1320
   tttcgttgta gtaacttaag ttatacagat gtcttctact gggtgggctt tctccatgag 1380
   tgggaggggg ctgggtgtca aataaaagtg tttctattaa aaaaaaaa 1428

<210> 14
<211> 372
<212> PRT
<213> Ixodes scapularis

<220>
<221> misc_feature
<222> (234)..(234)
<223> Xaa can be any naturally occurring amino acid

<400> 14

Arg Ala Val Gln Lys Leu Arg Arg Glu Val Glu Lys Ala Lys Arg Thr
1               5                   10                  15

Leu Ser Thr Ala His Gln Ala Arg Ile Glu Ile Glu Ser Phe Phe Glu
            20                  25                  30

Gly Glu Asp Phe Ser Glu Thr Leu Thr Arg Ala Lys Phe Glu Glu Leu
        35                  40                  45

Asn Met Asp Leu Phe Arg Ser Thr Met Lys Pro Val Gln Lys Val Leu
    50                  55                  60

Glu Asp Gly Asp Leu Lys Lys Thr Asp Val Asp Glu Ile Val Leu Val
65                  70                  75                  80

Gly Gly Ser Thr Arg Ile Pro Lys Val Gln Gln Leu Val Lys Glu Phe
                85                  90                  95

Phe Asn Gly Lys Glu Pro Thr Arg Gly Ile Asn Pro Asp Glu Ala Val
            100                 105                 110

Ala Tyr Gly Ala Ala Val Gln Ala Gly Val Leu Gly Gly Glu Glu Asp
        115                 120                 125

Thr Gly Asp Leu Val Leu Leu Asp Val Asn Pro Leu Thr Leu Gly Ile
    130                 135                 140

Glu Thr Val Gly Gly Val Met Thr Lys Leu Ile Pro Arg Asn Thr Val
145                 150                 155                 160

Ile Pro Thr Lys Lys Ser Gln Ile Phe Ser Thr Ala Ser Asp Glu Gln
                165                 170                 175

Ser Thr Val Thr Ile Gln Val Phe Glu Gly Glu Arg Pro Leu Thr Lys
            180                 185                 190

Asp Asn His Gln Leu Gly Lys Phe Asp Leu Thr Gly Ile Pro Pro Ala
        195                 200                 205

Pro Arg Gly Val Pro Gln Ile Glu Val Thr Phe Glu Ile Asp Val Asn
    210                 215                 220

Gly Ile Leu Arg Val Ser Ala Glu Asp Xaa Gly Thr Gly Asn Lys Gln
225                 230                 235                 240

Lys Ile Thr Ile Asn Asn Asp Gln Asn Arg Leu Thr Pro Glu Asp Ile
                245                 250                 255

Glu Arg Met Val Lys Asp Ala Glu Lys Phe Ala Asp Glu Asp Lys Lys
            260                 265                 270

Val Lys Glu Lys Val Glu Ala Arg Asn Glu Leu Glu Ser Tyr Ala Tyr
        275                 280                 285

Ser Leu Lys Asn Gln Ile Gly Asp Lys Glu Lys Met Gly Gly Lys Leu
    290                 295                 300

Ser Asp Glu Asp Lys Lys Thr Ile Glu Gln Ala Val Asp Glu Lys Ile
305                 310                 315                 320

Lys Trp Leu Glu Gln His Ser Asp Ala Asp Ala Glu Glu Leu Lys Glu
                325                 330                 335

Gln Lys Lys Gln Leu Ala Asp Thr Val Gln Pro Ile Val Ala Lys Leu
            340                 345                 350

Tyr Pro Ala Gly Gly Thr Pro Pro Pro Thr Asp Lys Asp Asp Ser Thr
        355                 360                 365

Lys Asp Glu Leu
    370

<210> 15
<211> 1847
<212> DNA
<213> Ixodes scapularis

<220>
<221> misc_feature
<222> (1814)..(1814)
<223> n is a, c, g, or t

<400> 15
cgacgtgttt gtgagtgcag cggtgaactg gacggtgtcg tggccacgcg atggcagcgg 60

cggtgatgaa ctgcctacgg actgcgcttt taggcgctct cgtcgtccaa ctctacgcca 120

cgcagatagg tcaccggaaa ttcgagtaca agtacagttt caagggaccc tacctggcgc 180

agaaggatgg atcggtgcct ttctgggagt acggcggcaa ttgcatcgcc agtgaggaga 240

tggttcggat cacgccctcc ctgaagagca agaaaggatc catctggtcc aagctgccga 300

catcgttccc ttggtgggag gtggagctgg tgttccgcac cacgggtacg ggcaggatag 360

gagctgacgg cctggccttc tggtacacag acaagaagca ggcggagggt cctgtctttg 420

gaagcagcga caagtggact ggcctggcca tcttcttcga ttccttcgac aatgataaca 480

agcacaacaa cccatacatc atgggcatgg tgaacgatgg aacaaaagcc tacgatcatg 540

agagtgacgg tgccaaccaa cagctagcgg gatgccagcg ggacttccgc aacaagcctt 600

accctgtcag ggccaagata gaatacttca acaacattct cacggtgctg ttccacaacg 660

gcaacaccaa caacgacggt gactacgaga tgtgcttccg tgcggagaac gtgttcctgc 720

cgaccaacgg ccactttggg gtgtccgccg ccacgggggg cctggcagac gaccacgacg 780

ccctcaagtt cctgacgacg agcctgcatg cggagggcac gcagccggcc ctggcccagg 840

gtatggccga ctcagagaag gagaagttct ccaaggagta tgaagtatac aaggacaagc 900

tggaaaagca gaaggaggag taccggaaga cgcacccgga ggaggccgct aagcaggcca 960

tggagcacgg ccccgagcag gcctacgaca cgcagcagca gcgcgagctg cgccagatct 1020

tcgagggcca gagccacaaa ttgtttgagg ggctcaaggc actgcaccgc aagctggacg 1080

aggtgctcgg gcgccaggag cgcaccctgt cgctggtgtc ggctggcggc gccggcgtgg 1140

ccgtgggcgg tgttccgcca ccgcagatgg gtggagtgcc gtcgctgcag aggcacgaag 1200

cagagtccct gctgagcagc cagcgggagc tgctgcagac ggtggctcag gtcaagagct 1260

ttgtggccga ggtgcatcaa cgcacggcca ccctgcaaca ccagggggcg ggaggcaccc 1320

agggcctcac ggccgagcag ctgcaagtgc tccaccaggt gcgggacagc gtggccagca 1380

tgcaccggga cgtctccaac aaccagccgc agaggactgg ctgcgcgaca tcctgtctca 1440

gcactaccca cttcttgctg tttgcaacgt tgcagttggc tgtcacgctg ggctacttgg 1500

tgtacaggag cagcaaagag gcggcggcca agaagttcta ctgagtgcag atctcgagcc 1560

ttgccttgcc ctcccctccc atggagtgga ccttaacccc acagactgcc agaaaccagt 1620

gttgccagag gagcccccct cccttcttat tgggtggggt gccacagcca tcacccattc 1680

ttcgagacaa ggccactgtt tggggggagg ggcaagagat tcatccgggg tgcgcaacaa 1740

aacatggccg tacagaggga ggggtgctcc agaactgggt cccagccaca tcgttgcgtg 1800

ggagcgcctt tctncctcac tctaaaaaa aaaaaaaaa aaaaaa 1847

<210> 16
<211> 497
<212> PRT
<213> Ixodes scapularis

<400> 16

Met Ala Ala Ala Val Met Asn Cys Leu Arg Thr Ala Leu Leu Gly Ala
1               5                   10                  15

Leu Val Val Gln Leu Tyr Ala Thr Gln Ile Gly His Arg Lys Phe Glu
            20                  25                  30

Tyr Lys Tyr Ser Phe Lys Gly Pro Tyr Leu Ala Gln Lys Asp Gly Ser
        35                  40                  45

Val Pro Phe Trp Glu Tyr Gly Gly Asn Cys Ile Ala Ser Glu Glu Met
    50                  55                  60

Val Arg Ile Thr Pro Ser Leu Lys Ser Lys Lys Gly Ser Ile Trp Ser
65                  70                  75                  80

Lys Leu Pro Thr Ser Phe Pro Trp Trp Glu Val Glu Leu Val Phe Arg
                85                  90                  95

Thr Thr Gly Thr Gly Arg Ile Gly Ala Asp Gly Leu Ala Phe Trp Tyr
            100                 105                 110

Thr Asp Lys Lys Gln Ala Glu Gly Pro Val Phe Gly Ser Ser Asp Lys
        115                 120                 125

Trp Thr Gly Leu Ala Ile Phe Phe Asp Ser Phe Asp Asn Asp Asn Lys
    130                 135                 140

His Asn Asn Pro Tyr Ile Met Gly Met Val Asn Asp Gly Thr Lys Ala
145                 150                 155                 160

Tyr Asp His Glu Ser Asp Gly Ala Asn Gln Gln Leu Ala Gly Cys Gln
                165                 170                 175

Arg Asp Phe Arg Asn Lys Pro Tyr Pro Val Arg Ala Lys Ile Glu Tyr
            180                 185                 190

Phe Asn Asn Ile Leu Thr Val Leu Phe His Asn Gly Asn Thr Asn Asn
        195                 200                 205

Asp Gly Asp Tyr Glu Met Cys Phe Arg Ala Glu Asn Val Phe Leu Pro

210                 215                 220

Thr Asn Gly His Phe Gly Val Ser Ala Ala Thr Gly Gly Leu Ala Asp
225                 230                 235                 240

Asp His Asp Ala Leu Lys Phe Leu Thr Thr Ser Leu His Ala Glu Gly
                245                 250                 255

Thr Gln Pro Ala Leu Ala Gln Gly Met Ala Asp Ser Glu Lys Glu Lys
            260                 265                 270

Phe Ser Lys Glu Tyr Glu Val Tyr Lys Asp Lys Leu Glu Lys Gln Lys
        275                 280                 285

Glu Glu Tyr Arg Lys Thr His Pro Glu Glu Ala Ala Lys Gln Ala Met
    290                 295                 300

Glu His Gly Pro Glu Gln Ala Tyr Asp Thr Gln Gln Gln Arg Glu Leu
305                 310                 315                 320

Arg Gln Ile Phe Glu Gly Gln Ser His Lys Leu Phe Glu Gly Leu Lys
                325                 330                 335

Ala Leu His Arg Lys Leu Asp Glu Val Leu Gly Arg Gln Glu Arg Thr
            340                 345                 350

Leu Ser Leu Val Ser Ala Gly Gly Ala Gly Val Ala Val Gly Gly Val
        355                 360                 365

Pro Pro Pro Gln Met Gly Gly Val Pro Ser Leu Gln Arg His Glu Ala
    370                 375                 380

Glu Ser Leu Leu Ser Ser Gln Arg Glu Leu Leu Gln Thr Val Ala Gln
385                 390                 395                 400

Val Lys Ser Phe Val Ala Glu Val His Gln Arg Thr Ala Thr Leu Gln
                405                 410                 415

His Gln Gly Ala Gly Gly Thr Gln Gly Leu Thr Ala Glu Gln Leu Gln
            420                 425                 430

Val Leu His Gln Val Arg Asp Ser Val Ala Ser Met His Arg Asp Val
        435                 440                 445

Ser Asn Asn Gln Pro Gln Arg Thr Gly Cys Ala Thr Ser Cys Leu Ser
    450                 455                 460

Thr Thr His Phe Leu Leu Phe Ala Thr Leu Gln Leu Ala Val Thr Leu
465                 470                 475                 480

Gly Tyr Leu Val Tyr Arg Ser Ser Lys Glu Ala Ala Ala Lys Lys Phe

485                 490                 495

Tyr

<210>  17
<211>  2475
<212>  DNA
<213>  Ixodes scapularis

<220>
<221>  misc_feature
<222>  (1342)..(1342)
<223>  n is a, c, g, or t

<220>
<221>  misc_feature
<222>  (1388)..(1388)
<223>  n is a, c, g, or t

<400>  17
catcactagt agcgagacac gtgcgtaaaa atggggccca aaacgctgtc taagcagccc      60

gctaaagctt cttcatccac ttccaagcgc accgccggcc ccacaataag caagcagacg     120

gaggacagcg atgacgaagg gtcaagcagc gcctactccg acttggagga ctccgaagga     180

gccgacagca gcgactcgaa cgatttgtcg gacacggagg cgtcggagga tgactacgat     240

gactcccaag acgaagaaaa cacgaagatt actttgactg gggtggaggg gaaggacctt     300

gagttgaggg ggaaggacca ggaggcaccg gtggagtctg gcaaaaggtc ggcatggcac     360

cggcagcaag aggacgccaa ggaggacaga cgaacgcaag tggtggaaga tgaatatgcc     420

tttgactctt ccgacgaaga ggacgttcgc aacacggttg gcaacattcc tctggagtgg     480

tacgagcact atccgcacat cggttatgat ctggaaggca agccaatcct gaagccgcct     540

cgggttagtg acctggacga cttcctgagg aaaatggatg accccaacta ttggaggacg     600

gtgaaggaca agagcacggg acaggacgtt gtcctgaccg acgaagatgt ggacctgatt     660

cagaggctgc agaaaggaca gttccccagc tcgacgactg acccttacga gccatttgag     720

gacatctttt cgcacgagac catgatccac ccggtgacca ggcaccctcc ccagaaacgc     780

agcttcgtgc cttcaaggat agaaaaagca atggtgtcaa agatggtgca cgcaatcaag     840

atgggctgga tcaagccccg agtaaagaag catgacccag aaagattcag cctcctgtgg     900

gacaaggatg actcgacagc gggcagcaat gagcgaatgc agcgccacat cccggcaccc     960

aagatgaagc tgccgggtca tgaggagtct tacaacccgc cggccgaata cctcttcacc    1020

gaggaagagg aggccaagtg gagagagcag gagcccgaag aacggcgcat aaacttcctg    1080

cccgccaagt acccatgtct gcgcgcagtc ccagcctacg aacgcttcat tgaggagagg    1140

tttgagcgct gtctggatct ctacttgtgc ccgaggcagc ggaagatgag ggtgaatgtg    1200

gatgcagagg acctgattcc tcagctgccc aaacccaagg acctgcagcc tttcccaagc    1260

attcagtcta ttgtctatga gggtcatacg gactgtgtcc tctgcctgtc tttggagcct    1320

gcgggacagt tctttgcatc anggtccgag gacggcaccg ttcgcatttg ggagctcttg 1380

acgggcangt gcctcaagaa gttccagttc gaggcgcccg tgaagagcgt ggcctggtgt 1440

ccagttgtcg ttcccatgaa actctgcgtg gacaagactg tttccatgct ggatgccgga 1500

gttacggaca aactgctgcc gttcaccacg ggacaccgag ttgtctgccc tccccgaaga 1560

gtcctcgggc caggcggcgg tagtggagtg ggagcagacg tcggcctcct ctccagagtt 1620

cctctcccgg ggggagcgtc tgcgggtcgt tcaccgccac ggtgtggtgc aggtgacgtg 1680

gcactcgagg ggagactact ttgccactgt cacggacgag ggacaggcca ccgtgcttgt 1740

ccatcagttg tccacgcggc ggttcgcagg ctccccttca gcaaggcgaa gggcggggtg 1800

tcccgggtgc tgttccaccc gctgcgcccc ttcctgctgg tggcgtgcca gcgcacagtg 1860

cgggtctacc acctgctcaa gcaggagctg gccaagaggc tcacatccaa ttgcaagtgg 1920

atctcgtgca tgggccgtcc acccccaggt gacaatctgc tgatcggcac gtacgagaag 1980

cggctgatgt ggttcgatct ggacctctcg accaaaccgt accagcagct gcgcatacac 2040

aatgccgcca tccgcagtgt ggcgttccat ccgcgctatc cactgtttgc gtccgccggc 2100

gacgatcgca gcgtgatcgt ttcgcacggt atggtgtaca atgatttact gcaaaaccca 2160

ctgatcgtgc cactgagacg gctgaagaac catgccatca gcaagggtat gggtgtgttg 2220

gactgcgcct tccatcccca ccagccgtgg atagtcacgg ccggagcaga cagcacgctg 2280

cggctcttca cctaagccgg gacgtcgtct ggtgtacata gtgaatcgtc aagaccgtgc 2340

caataaaagg actccacacc taaaaaaaaa aaaaaaaaaa aaaaaaaaaa aaaaaaaaaa 2400

aaaaaaaaaa aaaaaaaaaa aaaaaaaaaa aaaaaaaaaa aaaaaaaaaa aaaaaaaaaa 2460

aaaaaaaaaa aaaaa 2475

<210> 18
<211> 754
<212> PRT
<213> Ixodes scapularis

<220>
<221> misc_feature
<222> (438)..(438)
<223> Xaa can be any naturally occurring amino acid

<220>
<221> misc_feature
<222> (453)..(453)
<223> Xaa can be any naturally occurring amino acid

<400> 18

Met Gly Pro Lys Thr Leu Ser Lys Gln Pro Ala Lys Ala Ser Ser Ser
1               5                   10                  15

Thr Ser Lys Arg Thr Ala Gly Pro Thr Ile Ser Lys Gln Thr Glu Asp
            20                  25                  30

Ser Asp Asp Glu Gly Ser Ser Ser Ala Tyr Ser Asp Leu Glu Asp Ser

35                  40                  45

Glu Gly Ala Asp Ser Ser Asp Ser Asn Asp Leu Ser Asp Thr Glu Ala
    50                  55                  60

Ser Glu Asp Asp Tyr Asp Asp Ser Gln Asp Glu Glu Asn Thr Lys Ile
65                  70                  75                  80

Thr Leu Thr Gly Val Glu Gly Lys Asp Leu Glu Leu Arg Gly Lys Asp
                85                  90                  95

Gln Glu Ala Pro Val Glu Ser Gly Lys Arg Ser Ala Trp His Arg Gln
            100                 105                 110

Gln Glu Asp Ala Lys Glu Asp Arg Arg Thr Gln Val Val Glu Asp Glu
        115                 120                 125

Tyr Ala Phe Asp Ser Ser Asp Glu Glu Asp Val Arg Asn Thr Val Gly
    130                 135                 140

Asn Ile Pro Leu Glu Trp Tyr Glu His Tyr Pro His Ile Gly Tyr Asp
145                 150                 155                 160

Leu Glu Gly Lys Pro Ile Leu Lys Pro Pro Arg Val Ser Asp Leu Asp
                165                 170                 175

Asp Phe Leu Arg Lys Met Asp Asp Pro Asn Tyr Trp Arg Thr Val Lys
            180                 185                 190

Asp Lys Ser Thr Gly Gln Asp Val Val Leu Thr Asp Glu Asp Val Asp
        195                 200                 205

Leu Ile Gln Arg Leu Gln Lys Gly Gln Phe Pro Ser Ser Thr Thr Asp
    210                 215                 220

Pro Tyr Glu Pro Phe Glu Asp Ile Phe Ser His Glu Thr Met Ile His
225                 230                 235                 240

Pro Val Thr Arg His Pro Pro Gln Lys Arg Ser Phe Val Pro Ser Arg
                245                 250                 255

Ile Glu Lys Ala Met Val Ser Lys Met Val His Ala Ile Lys Met Gly
            260                 265                 270

Trp Ile Lys Pro Arg Val Lys Lys His Asp Pro Glu Arg Phe Ser Leu
        275                 280                 285

Leu Trp Asp Lys Asp Asp Ser Thr Ala Gly Ser Asn Glu Arg Met Gln
    290                 295                 300

Arg His Ile Pro Ala Pro Lys Met Lys Leu Pro Gly His Glu Glu Ser

305                 310                 315                 320

Tyr Asn Pro Pro Ala Glu Tyr Leu Phe Thr Glu Glu Glu Glu Ala Lys
                325                 330                 335

Trp Arg Glu Gln Glu Pro Glu Glu Arg Arg Ile Asn Phe Leu Pro Ala
            340                 345                 350

Lys Tyr Pro Cys Leu Arg Ala Val Pro Ala Tyr Glu Arg Phe Ile Glu
        355                 360                 365

Glu Arg Phe Glu Arg Cys Leu Asp Leu Tyr Leu Cys Pro Arg Gln Arg
    370                 375                 380

Lys Met Arg Val Asn Val Asp Ala Glu Asp Leu Ile Pro Gln Leu Pro
385                 390                 395                 400

Lys Pro Lys Asp Leu Gln Pro Phe Pro Ser Ile Gln Ser Ile Val Tyr
                405                 410                 415

Glu Gly His Thr Asp Cys Val Leu Cys Leu Ser Leu Glu Pro Ala Gly
            420                 425                 430

Gln Phe Phe Ala Ser Xaa Ser Glu Asp Gly Thr Val Arg Ile Trp Glu
        435                 440                 445

Leu Leu Thr Gly Xaa Cys Leu Lys Lys Phe Gln Phe Glu Ala Pro Val
    450                 455                 460

Lys Ser Val Ala Trp Cys Pro Val Val Val Pro Met Lys Leu Cys Val
465                 470                 475                 480

Asp Lys Thr Val Ser Met Leu Asp Ala Gly Val Thr Asp Lys Leu Leu
                485                 490                 495

Pro Phe Thr Thr Gly His Arg Val Val Cys Pro Pro Arg Arg Val Leu
            500                 505                 510

Gly Pro Gly Gly Gly Ser Gly Val Gly Ala Asp Val Gly Leu Leu Ser
        515                 520                 525

Arg Val Pro Leu Pro Gly Gly Ala Ser Ala Gly Arg Ser Pro Pro Arg
    530                 535                 540

Cys Gly Ala Gly Asp Val Ala Leu Glu Gly Arg Leu Leu Cys His Cys
545                 550                 555                 560

His Gly Arg Gly Thr Gly His Arg Ala Cys Pro Ser Val Val His Ala
                565                 570                 575

Ala Val Arg Arg Leu Pro Phe Ser Lys Ala Lys Gly Gly Val Ser Arg

            580                 585                 590

Val Leu Phe His Pro Leu Arg Pro Phe Leu Leu Val Ala Cys Gln Arg
        595                 600                 605

Thr Val Arg Val Tyr His Leu Leu Lys Gln Glu Leu Ala Lys Arg Leu
    610                 615                 620

Thr Ser Asn Cys Lys Trp Ile Ser Cys Met Gly Arg Pro Pro Pro Gly
625                 630                 635                 640

Asp Asn Leu Leu Ile Gly Thr Tyr Glu Lys Arg Leu Met Trp Phe Asp
                645                 650                 655

Leu Asp Leu Ser Thr Lys Pro Tyr Gln Gln Leu Arg Ile His Asn Ala
            660                 665                 670

Ala Ile Arg Ser Val Ala Phe His Pro Arg Tyr Pro Leu Phe Ala Ser
        675                 680                 685

Ala Gly Asp Asp Arg Ser Val Ile Val Ser His Gly Met Val Tyr Asn
    690                 695                 700

Asp Leu Leu Gln Asn Pro Leu Ile Val Pro Leu Arg Arg Leu Lys Asn
705                 710                 715                 720

His Ala Ile Ser Lys Gly Met Gly Val Leu Asp Cys Ala Phe His Pro
                725                 730                 735

His Gln Pro Trp Ile Val Thr Ala Gly Ala Asp Ser Thr Leu Arg Leu
            740                 745                 750

Phe Thr

<210> 19
<211> 447
<212> DNA
<213> Ixodes scapularis

<400> 19
caaagatgct gctgttctgc ccgacgtgcg ccaacatcct cattgtggaa caaggcttgg 60

agtgcttccg tttcgcctgc aacacatgcc cctacgtgca caacatcaag gcgaagatgt 120

cgaatcggaa gtacccgcgg ctcaaggacg tggacgacgt gctcggcggt gcagccgcct 180

gggagaatgt tgactcgacc gaagagaagt gccccaagtg tggccatgag cgggcctatt 240

ttatgcagat ccagactagg tcggccgacg agcccatgac caccttctac aagtgctgca 300

accagctctg tggccaccag tggagggact gacagatggc ggctttgacg aactcatgcc 360

cgtgcaaaat gcgtcggggg gagagagttt tggaataaaa catgcgcctt actttcataa 420

aaaaaaaaaa aaaaaaaaaa aaaaaaa 447

<210> 20
<211> 108
<212> PRT
<213> Ixodes scapularis

<400> 20

Met Leu Leu Phe Cys Pro Thr Cys Ala Asn Ile Leu Ile Val Glu Gln
1               5                   10                  15

Gly Leu Glu Cys Phe Arg Phe Ala Cys Asn Thr Cys Pro Tyr Val His
            20                  25                  30

Asn Ile Lys Ala Lys Met Ser Asn Arg Lys Tyr Pro Arg Leu Lys Asp
        35                  40                  45

Val Asp Asp Val Leu Gly Gly Ala Ala Ala Trp Glu Asn Val Asp Ser
    50                  55                  60

Thr Glu Glu Lys Cys Pro Lys Cys Gly His Glu Arg Ala Tyr Phe Met
65                  70                  75                  80

Gln Ile Gln Thr Arg Ser Ala Asp Glu Pro Met Thr Thr Phe Tyr Lys
                85                  90                  95

Cys Cys Asn Gln Leu Cys Gly His Gln Trp Arg Asp
            100                 105

<210> 21
<211> 1567
<212> DNA
<213> Ixodes scapularis

<220>
<221> misc_feature
<222> (785)..(785)
<223> n is a, c, g, or t

<400> 21
ccccccaggc gcagggcttc gttcaggtcg accagggggc cctccccgca agccccgagg 60

agcgccacct ggcaagcatg caggtcaatg gatatgagaa ccccacctac aagtacttcg 120

aggccaacac caactgagcg gccacgcccc caggggaggg ggaaaagggg gcggacggac 180

gtattgtgcc tgctgcgggc tgcgggatta gctcgtcccg cgttgttccg ggagccagtt 240

ggtttgcctc gcgtcttagg agtaggcacg gcctcccttc tgcacccggt caaggaccat 300

ggttgttggg gacacgagcg gcgtggggcg cagccagcct gagctttggg tcccggtacc 360

acggcaaacc gtttgttccc acccgcggaa tgaaaatttt gtttgcctca gtttctttcg 420

aatcgagcgt cggcgccgcc tccgacagcc ccgagtgcac tctgtctgtt gcgaaagacc 480

aatggagtag ttgacactcg ggtcgcagct cgaacaagct cccgtaaaac gctacttaac 540

cggggccggc gaccgagcgt agagcttgct gtgcgtagtt gtggataaaa cttttttttt 600

ttgtgtgtgt gcttggtcac agacaatggg cagcttccga cgttagccac gcgccacacg 660

ctcgcctttg ttttcttctt ctcgcggttg tcatacttag tttccattgg cgggttaaca 720

ttccagtccg ggcgggcgcc cccgttcagg cgcgtcctga tcaaaattga gcatttggtt 780

gtgcngtgca tttattggcc gcagcagggg gttcccgggt gcacctggtg tcgtgacacg 840

catgtcgtga ctttcccctc agacggttgt ccttgctcat ggctcgttca cacctctagt 900

gctggtagtc tctgttgctt aggtttgtag gagcacacta cagcagaggg tgtcacaaag 960

ttttctaagc tgtatataca tgaggaaaac attgcgttgc acacacgcga gtttcggcct 1020

gtttttagtt gggacagtga acgttttttg tacaggttat tatgtagtgc ctacatttgt 1080

atgtgccagc tgcatgtgtt ttcctgcatg tggggaagcc tccgtgctgc cccgagctgt 1140

gtgcggcccc tcctgagttt ccatgtgcca tgtgcccagc ctagggtgaa ctgggggtgc 1200

agatgccctt gcgcacggtg tgccccggcg agcattgtgt gtccgtaggc catcgacgct 1260

attcatgcga aattaatgtg gtcacagctg tcattgtctc agtgaacata tcatatgtcc 1320

aaatttgtct cccctgtcag tgtgtgcttc tcttggttct acacttgcct gcatttttgt 1380

tagtttgccg gactgtcctt ttcggtccca ggtcgacagc aggctataac aacaattccg 1440

gtattttcca gtatcgggtc acaccaggtg taacctattg tgcatgtagt gtaacttgag 1500

tggaaaagct aaaataaaaa tttgcaagag tctcactaaa aaaaaaaaaa aaaaaaaaaa 1560

aaaaaaa 1567

<210> 22
<211> 44
<212> PRT
<213> Ixodes scapularis

<400> 22

Pro Gln Ala Gln Gly Phe Val Gln Val Asp Gln Gly Ala Leu Pro Ala
1               5                   10                  15

Ser Pro Glu Glu Arg His Leu Ala Ser Met Gln Val Asn Gly Tyr Glu
            20                  25                  30

Asn Pro Thr Tyr Lys Tyr Phe Glu Ala Asn Thr Asn
        35                  40

<210> 23
<211> 704
<212> DNA
<213> Ixodes scapularis

<220>
<221> misc_feature
<222> (534)..(534)
<223> n is a, c, g, or t

<220>
<221> misc_feature

<222>  (598)..(598)
<223>  n is a, c, g, or t

<400>  23
tgagaagaca ctagaggaca agttcttcga gcatgaggtg atgctgaatg tgaatgcgtt      60

catgcagcag ttccattccg gcgtttttta tgcctacgtg aagctgaagg aacaagagtg     120

ccgcaacatt gtctggattg ccgaatgcgt tgctcagcgt catcggtcca agatcgataa     180

ctacattcca atcttctagt cgctcgagga aaagaaatgg gccaattcgg tagtttgtcg     240

gtgtaatata tatatatata tatatctact tcgcaaaatt cttcagctag agtgtctatg     300

tctggttagc tgcgattgtg cgagagggga aaaaaatgta gtcagtggca tgatcaagga     360

aggaaaaaaa ttggccaata acttttacct tttgaagtta aagcaagggt taaaataatg     420

tctattttta cttcgcttta ccgtgtgctg gctattgctt tgcaaacgtt ttttaaaatt     480

tttgcagttc gtctttcttc ttttgagcac atatttattc cagagttcca atancctttt     540

atgtgtgaat gaatgactaa tccatgttgg ggttggttaa tggtgcattg ttgaaaanat     600

aaaccccaac tccagctggc ctttggaaaa aaaaaaaaaa aaaaaaaaaa aaaaaaaaaa     660

aaaaaaaaaa aaaaaaaaaa aaaaaaaaaa aaaaaaaaaa aaaa                      704


<210>  24
<211>  681
<212>  DNA
<213>  Ixodes scapularis


<220>
<221>  misc_feature
<222>  (432)..(432)
<223>  n is a, c, g, or t

<220>
<221>  misc_feature
<222>  (467)..(467)
<223>  n is a, c, g, or t

<220>
<221>  misc_feature
<222>  (472)..(472)
<223>  n is a, c, g, or t

<220>
<221>  misc_feature
<222>  (481)..(481)
<223>  n is a, c, g, or t

<220>
<221>  misc_feature
<222>  (493)..(493)
<223>  n is a, c, g, or t

<220>
<221>  misc_feature
<222>  (495)..(495)
<223>  n is a, c, g, or t

<220>
<221>  misc_feature
<222>  (499)..(499)

<223>  n is a, c, g, or t

<220>
<221>  misc_feature
<222>  (507)..(507)
<223>  n is a, c, g, or t

<220>
<221>  misc_feature
<222>  (515)..(515)
<223>  n is a, c, g, or t

<220>
<221>  misc_feature
<222>  (518)..(518)
<223>  n is a, c, g, or t

<220>
<221>  misc_feature
<222>  (528)..(528)
<223>  n is a, c, g, or t

<220>
<221>  misc_feature
<222>  (533)..(533)
<223>  n is a, c, g, or t

<220>
<221>  misc_feature
<222>  (547)..(547)
<223>  n is a, c, g, or t

<220>
<221>  misc_feature
<222>  (550)..(550)
<223>  n is a, c, g, or t

<220>
<221>  misc_feature
<222>  (559)..(559)
<223>  n is a, c, g, or t

<220>
<221>  misc_feature
<222>  (565)..(565)
<223>  n is a, c, g, or t

<220>
<221>  misc_feature
<222>  (567)..(567)
<223>  n is a, c, g, or t

<220>
<221>  misc_feature
<222>  (571)..(571)
<223>  n is a, c, g, or t

<220>
<221>  misc_feature
<222>  (586)..(586)
<223>  n is a, c, g, or t

<220>
<221>  misc_feature
<222>  (593)..(593)
<223>  n is a, c, g, or t

<220>

<221> misc_feature
<222> (599)..(599)
<223> n is a, c, g, or t

<220>
<221> misc_feature
<222> (603)..(603)
<223> n is a, c, g, or t

<220>
<221> misc_feature
<222> (606)..(606)
<223> n is a, c, g, or t

<220>
<221> misc_feature
<222> (611)..(611)
<223> n is a, c, g, or t

<220>
<221> misc_feature
<222> (619)..(619)
<223> n is a, c, g, or t

<220>
<221> misc_feature
<222> (623)..(623)
<223> n is a, c, g, or t

<220>
<221> misc_feature
<222> (625)..(625)
<223> n is a, c, g, or t

<220>
<221> misc_feature
<222> (627)..(627)
<223> n is a, c, g, or t

<220>
<221> misc_feature
<222> (651)..(651)
<223> n is a, c, g, or t

<220>
<221> misc_feature
<222> (658)..(658)
<223> n is a, c, g, or t

<220>
<221> misc_feature
<222> (666)..(666)
<223> n is a, c, g, or t

<220>
<221> misc_feature
<222> (673)..(674)
<223> n is a, c, g, or t

<400> 24
gtcacgggat ttgggaagct gtcgtctgtc gtcctgcagt ttcaaacggt ttcaccaaaa 60

acctttccgt ctcgctgtca gacgccttga accatgactg agttctggct catctcggct 120

ccgggcgaga aaacctgcca acagacttat gacaagctgc tcagcgtcac aagcaacaag 180

cagaacaacc tctcgacctg ctacaagttc caccttccgg acttgaaggt gggtacgctg 240

gatcagttgg ttggcctctc ggatgacttg ggaaagctcg acacctatgt cgaaagcatc     300

actcgaaaag tggccagcta tctgggggac gtgcttgacg accagaggga caaactagcc     360

gacaaccttc cttgccaatg gcttggggct ggaggcctac ctgaccccgg ttttcagtgg     420

gacatggcca antaccccat caagcagttc gcctcaagag catcacntga antcatcagc     480

nagcaagtgt ctnanattng accggtngaa cctcnagnag caagttanct tgnttacaac     540

aaccttnaan aacttaagnt tcaantncat ncgaacccca aatccnccgg ggnaggccng     600

gcnttnttcc ngttagccnt ggncntnacc ttattgcgcc aagggagcca ntttgtcntt     660

gggggntcgg ganntacctt a                                               681

<210>  25
<211>  720
<212>  DNA
<213>  Ixodes scapularis

<220>
<221>  misc_feature
<222>  (488)..(488)
<223>  n is a, c, g, or t

<220>
<221>  misc_feature
<222>  (625)..(625)
<223>  n is a, c, g, or t

<220>
<221>  misc_feature
<222>  (627)..(627)
<223>  n is a, c, g, or t

<220>
<221>  misc_feature
<222>  (631)..(631)
<223>  n is a, c, g, or t

<220>
<221>  misc_feature
<222>  (641)..(641)
<223>  n is a, c, g, or t

<220>
<221>  misc_feature
<222>  (680)..(680)
<223>  n is a, c, g, or t

<220>
<221>  misc_feature
<222>  (692)..(692)
<223>  n is a, c, g, or t

<220>
<221>  misc_feature
<222>  (700)..(700)
<223>  n is a, c, g, or t

<220>
<221>  misc_feature
<222>  (719)..(719)
<223>  n is a, c, g, or t

<400> 25
ctctcagcga ctccgacgtc caaaagcaga tcaagcacat gatggctttc atcgaccagg 60

aagccaacga aaaggcagaa gaagtagacg ccaaggcagg aagaagagtt caacatcgag 120

aagggccgcc tggtcacgga gcaaaggctc aagatcatcg actactacac ccgtcgagag 180

aagcaagttg aactgcagcg caagatccaa agctccaaca tgctgaacca ggcccggctg 240

aaggtgctga aggcgggcga ggaccacatt gcgacggtgc tggaggaggc caagcgccgc 300

ctgggggaca tcaccaggga ccaggctcgc taccaagccc tcctgcagag catggttctg 360

caggcactgc ttcagctcct cgagcaggag gtggtcgtcc actgccgacc gcaagacgcc 420

gggctgctga acttggacac gctgagtgcc aagttcaagg aggccactgg ccgagaggtc 480

aagctcantg tggagcccag cctggcttcg agcagctgcg gcggagtcga gatgctctcc 540

aggcggggca agattcgcgt ctgcaacacg ctcgagtcgc ggctggacat gattgccctt 600

cagctttctg ccgcagatca agacngncct nttcggcagg nacccccaac cgcaagttca 660

tggactaggc gggctattgn ccccgccatt cnggccagtn agcttggacc gtgtttacng 720

<210> 26
<211> 212
<212> DNA
<213> Dermacentor variabilis

<400> 26
atggcttgcg caacattaaa gcgaacacac gattgggatc cgctgcatag tccaaacgga 60

agatcgccca aacgacggag atgtatgcct ttgtcggtca cacaagcagc gactccccca 120

acaagggcac accaaatcaa cccttcaccc ttcggtgaag tgccaccgaa attaacttca 180

gaggagatag cggccaacat tcgggaggaa at 212

<210> 27
<211> 70
<212> PRT
<213> Dermacentor variabilis

<400> 27

Met Ala Cys Ala Thr Leu Lys Arg Thr His Asp Trp Asp Pro Leu His
1               5                   10                  15

Ser Pro Asn Gly Arg Ser Pro Lys Arg Arg Arg Cys Met Pro Leu Ser
            20                  25                  30

Val Thr Gln Ala Ala Thr Pro Pro Thr Arg Ala His Gln Ile Asn Pro
        35                  40                  45

Ser Pro Phe Gly Glu Val Pro Pro Lys Leu Thr Ser Glu Glu Ile Ala
    50                  55                  60

Ala Asn Ile Arg Glu Glu
65                  70

<210> 28
<211> 490
<212> DNA
<213> Amblyomma americanum

<400> 28
atggcttgcg caacattaaa gcgaacgcac gactgggatc ccctgcacag tccgaacgga 60

agatcaccaa agcggcgaag atgtatgcct ttttcagtca acccaccagc tcctccaaca 120

agggcgcacc aaatcaatcc ctcgcctttc ggcgacgtgc caccaaagct gacttcagag 180

gagatagcag ccaacatccg tgaggaaatg cggcggctgc agcggcgcaa gcagctctgc 240

ttccagggtg cagagtgcag tagccccccc gaaggctgtt cccccattgg ctccccctcc 300

cagcacaccg gcggcctcct gtcacctgtg cgccgagacc agcccctgtt caccttccgc 360

caagtgggac tcatctgtga gcgcatgatg aaagagcgag agagccagat acgagaggag 420

tatgaccacg tgctctccac caagcttgca gagcagtacg acacatttgt caagtttacg 480

tacgaccaaa 490

<210> 29
<211> 163
<212> PRT
<213> Amblyomma americanum

<400> 29

Met Ala Cys Ala Thr Leu Lys Arg Thr His Asp Trp Asp Pro Leu His
1               5                   10                  15

Ser Pro Asn Gly Arg Ser Pro Lys Arg Arg Arg Cys Met Pro Phe Ser
            20                  25                  30

Val Asn Pro Pro Ala Pro Pro Thr Arg Ala His Gln Ile Asn Pro Ser
        35                  40                  45

Pro Phe Gly Asp Val Pro Pro Lys Leu Thr Ser Glu Glu Ile Ala Ala
    50                  55                  60

Asn Ile Arg Glu Glu Met Arg Arg Leu Gln Arg Arg Lys Gln Leu Cys
65                  70                  75                  80

Phe Gln Gly Ala Glu Cys Ser Ser Pro Pro Glu Gly Cys Ser Pro Ile
                85                  90                  95

Gly Ser Pro Ser Gln His Thr Gly Gly Leu Leu Ser Pro Val Arg Arg
            100                 105                 110

Asp Gln Pro Leu Phe Thr Phe Arg Gln Val Gly Leu Ile Cys Glu Arg
        115                 120                 125

Met Met Lys Glu Arg Glu Ser Gln Ile Arg Glu Glu Tyr Asp His Val
    130                 135                 140

Leu Ser Thr Lys Leu Ala Glu Gln Tyr Asp Thr Phe Val Lys Phe Thr
145                 150                 155                 160

Tyr Asp Gln

<210>  30
<211>  442
<212>  DNA
<213>  Boophilus microplus

<400>  30
atggcttgcg caacattaaa gcgaacacat gactgggacc ccttgcacag tccgagtggc      60

agatcgccga agcgacgccg atgtatgcct ctgtccccgc ctcctacaag ggcgcaccag     120

atcgatccct cgcccttcgg agacgtgcca ccaaagttga cttcagagga gatagcagcc     180

aacatccgtg aggagatgcg acggctacaa cggcgcaagc agctctgttt ccagggcgct     240

gaccctgaat cccagcatac cagtggtctc tcgtcaccag tgcatcgaga ccagcccctg     300

ttcacgttcc gccaggttgg gctcatttgc gaacgaatga tgaaggagcg agagagcaag     360

atacgggagg agtatgacca tgtgctatct accaaactcg cagaacagta cgacacattt     420

gtcaagttta cgtacgacca aa                                              442

<210>  31
<211>  147
<212>  PRT
<213>  Boophilus microplus

<400>  31

Met Ala Cys Ala Thr Leu Lys Arg Thr His Asp Trp Asp Pro Leu His
1               5                   10                  15

Ser Pro Ser Gly Arg Ser Pro Lys Arg Arg Arg Cys Met Pro Leu Ser
            20                  25                  30

Pro Pro Pro Thr Arg Ala His Gln Ile Asp Pro Ser Pro Phe Gly Asp
        35                  40                  45

Val Pro Pro Lys Leu Thr Ser Glu Glu Ile Ala Ala Asn Ile Arg Glu
    50                  55                  60

Glu Met Arg Arg Leu Gln Arg Arg Lys Gln Leu Cys Phe Gln Gly Ala
65                  70                  75                  80

Asp Pro Glu Ser Gln His Thr Ser Gly Leu Ser Ser Pro Val His Arg
                85                  90                  95

Asp Gln Pro Leu Phe Thr Phe Arg Gln Val Gly Leu Ile Cys Glu Arg
            100                 105                 110

Met Met Lys Glu Arg Glu Ser Lys Ile Arg Glu Glu Tyr Asp His Val
        115                 120                 125

Leu Ser Thr Lys Leu Ala Glu Gln Tyr Asp Thr Phe Val Lys Phe Thr
    130                 135                 140

Tyr Asp Gln
145

<210>  32
<211>  23
<212>  DNA
<213>  Artificial Sequence

<220>
<223>  oligonucleotide primer

<400>  32
gcgtcgtgtg gagcatcagc gac                                              23

<210>  33
<211>  23
<212>  DNA
<213>  Artificial Sequence

<220>
<223>  oligonucleotide primer

<400>  33
tcgcaacgga caacggcagg ttg                                              23

<210>  34
<211>  23
<212>  DNA
<213>  Artificial Sequence

<220>
<223>  oligonucleotide primer

<400>  34
gcttgcgcaa cattaaagcg aac                                              23

<210>  35
<211>  23
<212>  DNA
<213>  Artificial Sequence

<220>
<223>  oligonucleotide primer

<400>  35
tgcttgtttg cagatgccca tca                                              23

<210>  36
<211>  23
<212>  DNA
<213>  Artificial Sequence

<220>
<223>  oligonucleotide primer

<400>  36
gaaatatctg tgaaaccaag gcc                                              23

<210> 37
<211> 23
<212> DNA
<213> Artificial Sequence

<220>
<223> oligonucleotide primer

<400> 37
attgcacaac acatcattaa ctg 23

<210> 38
<211> 486
<212> DNA
<213> Rhipicephalus appendiculatus

<400> 38
atggcttgtg cgacattaaa gcggacacat gactgggacc ccttgcacag tccgagtggc 60

agatcaccaa agcgacgccg atgcatgcct ctgtccccgc ctccaacgag ggcgcaccag 120

atcgatccct cgcccttcgg agacgtgcca ccaaagttga cttcagagga gatagcagcc 180

aacatccgcg aggagatgcg acggctacaa cggcgcaagc agctctgttt tcaaggcacc 240

gacgccgaat cccagcatac cagtggtctc tcgtcaccgg tgcgtcgaga ccagcccctg 300

ttcacgttcc gccaggttgg gctcatttgc gaacgcatga tgaaggagcg agagagcaag 360

atacgggagg agtatgacca cgtgctatcc accaagctcg cagaacagta cgacacgttt 420

gttaaattta cctacgacca aattcagaag cggtttgagg gtgctacgcc cagctatttg 480

tcgtaa 486

<210> 39
<211> 161
<212> PRT
<213> Rhipicephalus appendiculatus

<400> 39

Met Ala Cys Ala Thr Leu Lys Arg Thr His Asp Trp Asp Pro Leu His
1               5                   10                  15

Ser Pro Ser Gly Arg Ser Pro Lys Arg Arg Arg Cys Met Pro Leu Ser
            20                  25                  30

Pro Pro Pro Thr Arg Ala His Gln Ile Asp Pro Ser Pro Phe Gly Asp
        35                  40                  45

Val Pro Pro Lys Leu Thr Ser Glu Glu Ile Ala Ala Asn Ile Arg Glu
    50                  55                  60

Glu Met Arg Arg Leu Gln Arg Arg Lys Gln Leu Cys Phe Gln Gly Thr
65                  70                  75                  80

Asp Ala Glu Ser Gln His Thr Ser Gly Leu Ser Ser Pro Val Arg Arg
                85                  90                  95

Asp Gln Pro Leu Phe Thr Phe Arg Gln Val Gly Leu Ile Cys Glu Arg
            100                 105                 110

Met Met Lys Glu Arg Glu Ser Lys Ile Arg Glu Glu Tyr Asp His Val
        115                 120                 125

Leu Ser Thr Lys Leu Ala Glu Gln Tyr Asp Thr Phe Val Lys Phe Thr
    130                 135                 140

Tyr Asp Gln Ile Gln Lys Arg Phe Glu Gly Ala Thr Pro Ser Tyr Leu
145                 150                 155                 160

Ser

## Description

### CROSS REFERENCE TO RELATED APPLICATIONS

This application is a continuation-in-part of copending U.S. Patent Application Serial No. 10/425,563, filed April 29, 2003, which application claims the benefit of U.S. Provisional Patent Application Serial No. 60/376,251 filed April 29, 2002. Both noted priority applications are incorporated herein by reference.

### BACKGROUND OF THE INVENTION

### Technical Field:

The present invention relates to the identification of protective antigens against tick infestations, gene sequences and encoded proteins for such antigens, related vaccines and methods useful to induce an immune response in mammals, which are protective against a broad range of tick species. Background:

Ticks parasitize wild, domesticated animals and humans and transmit pathogens including fungi, bacteria, viruses and protozoon. Currently, ticks are considered to be second in the world to mosquitoes as vectors of human diseases, but they are considered to be the most important vector of pathogens in North America (Parola and Raoult, 2001). *Ixodes spp.* are distributed worldwide and act as vectors of human diseases caused by *Borrelia burgdorferi* (Lyme disease), *Anaplasma phagocytophila* (human granulocytic ehrlichiosis), *Coxiella burnetti* (Q fever), *Francisella tularensis* (tularemia), *B. afzelii, B. lusitaniae, B. valaisiana* and *B. garinii, Rickettsia helvetica, R. japonica* and *R. australis, Babesia divergens* and tick-borne encephalitis (TBE) and Omsk Hemorrhagic fever viruses (Estrada-Peña and Jongejan, 1999; Parola and Raoult, 2001). Throughout eastern and southeastern United States and Canada, *I. scapularis* (the black legged tick) is the main vector of *B. burgdorferi* sensu stricto and *A. phagocytophila* (Estrada-Peña and Jongejan, 1999; Parola and Raoult, 2001). Besides *Ixodes,* other tick species of medical and veterinary importance include those of the genera *Amblyomma, Dermacentor, Rhipicephalus* and *Boophilus.*

Control of tick infestations is difficult and often impractical for multi-host ticks such as *Ixodes* spp. Presently, tick control is effected by integrated pest management in which different control methods are adapted to one area or against one tick species with due consideration to their environmental effects. Control of ticks by vaccination, however, would avoid environmental contamination and selection of drug resistant ticks that result from repeated acaricide application (de la Fuente et al., 1998; Garcia-Garcia et al., 1999, de la Fuente and Kocan 2004). Anti-tick vaccines would also allow for inclusion of multiple antigens in order to target a broad range of tick species and for incorporation of pathogen-blocking antigens (de la Fuente and Kocan 2004).

Recently, vaccines have been developed against one-host *Boophilus spp*. (Willadsen, 1997; Willadsen and Jongejan, 1999; de la Fuente et al., 1999; 2000; de Vos et al., 2001; de la Fuente and Kocan 2004). The recombinant *B. microplus* BM86 gut antigen included in commercial vaccine formulations TickGARD (Hoechst Animal Health, Australia) and Gavac (Heber Biotec S. A., Havana, Cuba) also confers partial protection against phylogenetically related *Hyalomma* and *Rhipicephalus* tick genera (de la Fuente et al., 2000; de Vos et al., 2001; de la Fuente and Kocan 2004). However, immunization with BM86 failed to protect against the more phylogenetically distant *Amblyomma* spp. (de Vos et al., 2001). These results suggest that using Bm86 or a closely related gene for the production of vaccines against *Ixodes* spp. or other tick genera phylogenetically distant from *Boophilus* spp. (Black and Piesman, 1994) could be impractical. Therefore, there remains a need to identify broad range vaccine candidates against tick infestations across phylogenetically distant species (de la Fuente and Kocan 2004).

In parent application Serial No. 10/425,563 we described the identification by ELI and sequence analysis of protective cDNA clones against experimental infestations with *I. Scapularis* in what was the first example of the application of ELI to arthropods and particularly to ticks (Almazán et al 2003 a,b). It was thought that these protective antigens, although identified for *I. scapularis,* might be cross protective between *Ixodes* species considering the high degree of conservation of gene sequences and protein function between species of the same genus.

Surprisingly, it has been discovered that certain of the protective antigens exhibit activity and are expressed across tick species, and in some circumstances across genera, thus making possible the use of these common antigens in broad range anti-tick vaccine formulations.

### SUMMARY OF THE INVENTION

In the parent application, numerous protective antigens derived from *I. scapularis* were identified and shown to possess protective properties against *I. scapularis* tick infestations. Among these antigens were clones and associated polypeptides designated 4D8, 4F8 and 4E6. Herein the activity of these particular protective antigens is demonstrated to extend to other tick species, and, in the case of 4D8, 4F8 and 4E6 to non-*Ixodes* tick species. In addition, there is provided the recently discovered sequences (DNA and protein) of tick protective antigen 4D8 derived from genus Rhipicephalus, particularly Rhipicephalus appendiculatus.

Sequence conservation and expression of 4F8, 4D8 and 4E6 were analyzed in *I. scapularis* related species, *I. pacificus* and *I. ricinus,* and in *D. variabilis, R. sanguineus, B. microplus* and *A. americanum* by RT-PCR using the primers specific for the known *I. scapularis* sequences. Expression was detected in *I. ricinus* for all three genes and in *I. pacificus* for 4D8 and 4E6. Expression of 4D8 was also detected in *D. variabilis, B. microplus, R. sanguineus* and *A. americanum.* Corresponding 4D8 genes of *D. variabilis, A. americanum and B. microplus* were cloned and sequenced.

The detection of 4D8 and 4E6 expression in non-*Ixodes* tick species were the basis for an experiment in which rabbits were immunized with *I. scapularis* derived 4F8, 4D8 and 4E6 and challenged with *I. scapularis, D. variabilis* and *A. americanum* nymphs. Results support using the inventive protective antigens in vaccine formulations for the control of multiple tick species.

Accordingly, in one embodiment of the present invention there is provided cDNA sequences, protein encoding fragments thereof, and derived protein sequences for the multi-species protective antigens denominated 4F8, 4D8 and 4E6.

In another embodiment of the present invention there is provided a vaccine composition comprising the inventive protective recombinant proteins and/or modified cDNAs separately or which may optionally be combined with adjuvant to enhance the protection efficacy of vaccine preparations against multiple tick species, wherein the vaccine composition further comprises a pharmaceutically acceptable carrier or diluent. The vaccine composition also may optionally be combined with tick-borne pathogen components to provide a means to control tick-borne infections, wherein the vaccine composition further comprises a pharmaceutically acceptable carrier or diluent and adjuvant.

In another embodiment of the present invention there is provided a method for inducing an immune response in a mammal to provide immune protection, which reduces or affects infestations by target ticks and/or transmission of tick-borne pathogens, the method comprising administering to at-risk human population and mammalian reservoir an effective amount of a vaccine composition comprising the inventive protective recombinant proteins and/or modified cDNAs alone or in combination with an adjuvant or tick-borne pathogen components to provide a means to control tick infestations and to reduce transmission to humans of tick-borne infections, wherein the vaccine composition further comprises a pharmaceutically acceptable carrier or diluent.

A better understanding of the present invention and its objects and advantages will become apparent to those skilled in this art from the following detailed description, wherein there is described only the preferred embodiment of the invention, simply by way of illustration of the best mode contemplated for carrying out the invention. As will be realized, the invention is capable of modifications in various obvious respects, all without departing from the scope and spirit of the invention. Accordingly, the description should be regarded as illustrative in nature and not as restrictive.

### BRIEF DESCRIPTION OF THE DRAWINGS

FIG. 1 is a summary of the cDNA ELI approach used to identify protective antigens against *I. scapularis* infestations.

FIG. 2A is a graph depicting the results of a primary screen of cDNA pools (A-H 1-4, A5) by ELI. V, control mice injected with 1 µg vector DNA alone.
*α<0.01, **α<0.05 (Tukey's *post-hoc* test for pair comparisons after ANOVA). Number in boxes represent values for inhibition of tick infestation with respect to the control group.

FIG. 2B is a graph depicting the results of a primary screen of cDNA pools (A6-A10, B-H 5-8) by ELI. V, control mice injected with 1 µg vector DNA alone.
*α<0.01, **α<0.05 (Tukey's *post-hoc* test for pair comparisons after ANOVA). Number in boxes represent values for inhibition of tick infestation with respect to the control group.

FIG. 3 is a graph depicting the results of a tertiary screen by ELI of cDNA sub-pools formed according to the predicted function of encoded proteins. Only groups with I≥15% are shown (white bars). The number of engorged larvae per mouse is expressed as mean±SD (black bars). Control mice were injected with mitochondrial (MT) cDNAs. *P≤0.05 (Student's t-test).

### DETAILED DESCRIPTION OF THE INVENTION

Before explaining the present invention in detail, it is important to understand that the invention is not limited in its application to the details of the construction illustrated and the steps described herein. The invention is capable of other embodiments and of being practiced or carried out in a variety of ways. It is to be understood that the phraseology and terminology employed herein is for the purpose of description and not of limitation.

In the parent application, there were provided 25 separate and distinct sequences comprising 14 cloned cDNA molecules and 11 deduced amino acid sequences of encoded polypeptides, said sequences having been isolated and identified as protective against *I. scapularis* in accordance with the following described experimental methodology.

### Example 1: Construction of an I. scapularis cDNA library and screening for protective antigens by ELI

### Tick cells

Monolayers of IDE8 (ATCC CRL 1973) cells, originally derived from embryonic *I. scapularis,* were maintained at 31°C in L-15B medium supplemented with 5% foetal bovine serum, tryptose phosphate broth and bovine lipoprotein concentrate after Munderloh et al. (1994). Cells were subcultured at 1:5 - 1:10 when monolayers reached a density of approximately 10⁷ cells/T-25 flask. Medium was replaced weekly.

### Library construction

A cDNA expression library was constructed in the vector pEXP1 containing the strong cytomegalovirus CMV_{IE} promoter (Clontech). Because we planned to target the early larval stages of *I. scapularis,* we chose to construct our library from cultured embryonic *I. scapularis* IDE8 cells-derived poly(A)+ RNA. The cDNA library contained 4.4 x 10⁶ independent clones and a titer of approximately 10¹⁰ cfu/ml with more than 93% of the clones with cDNA inserts. The average cDNA size was 1.7 kb (0.5-4.0 kb).

### Primary screen

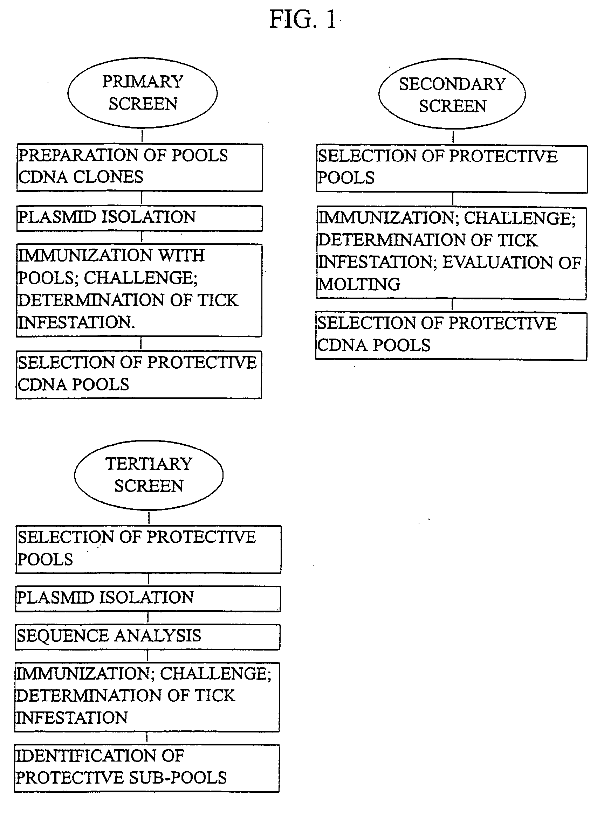

The overall schema for identification of protective antigens through ELI, sequential fractionation and sequence analysis is shown in Fig. 1.

Ninety-six LBA (master) plates containing an average of 41 (30-61) cDNA clones per plate were prepared. Replicas were made and clones from each plate were pooled, inoculated in Luria-Bertani with 50 µg/ml ampicillin, grown for 2 hr in a 96 wells plate and plasmid DNA purified from each pool (Wizard SV 96 plasmid DNA purification system, Promega, Madison, WI, USA). BALB/c female mice, 5-6 weeks of age at the time of first vaccination, were used. Mice were cared for in accordance with standards set in the Guide for Care and Use of Laboratory Animals. Mice were injected with a 1 ml tuberculin syringe and a 27 gauge needle at days 0 and 14. Three mice per group were each immunized IM in the thigh with 1 µg DNA/dose in 50 µl PBS. Two groups of 3 mice each were included as controls. One group was injected with 1 µg vector DNA alone and the second with saline only. Two weeks after the last immunization, mice were infested with 100 *I. scapularis* larvae per mouse. Ticks were artificially reared at the Oklahoma State University tick rearing facility by feeding larvae on mice, nymphs on rabbits and adults on sheep and using for infestation in our experiments the larvae obtained from the eggs oviposited by a single female. Twelve hours after tick infestation, larvae that did not attach were counted to calculate the number of attached larvae per mouse and mice were transferred to new cages. Replete larvae dropping from each mouse were collected daily and counted during 7 days. The inhibition of tick infestation (I) for each test group was calculated with respect to vector-immunized controls as [1-(<RL>n/<RL>c x <RL>ic/<RL>in)] x 100, where <RL>n is the average number of replete larvae recovered per mouse for each test group, <RL>c is the average number of replete larvae recovered per mouse for control group, <RL>ic is the average number of larvae attached per mouse for control group, and <RL>in is the average number of larvae attached per mouse for each test group.

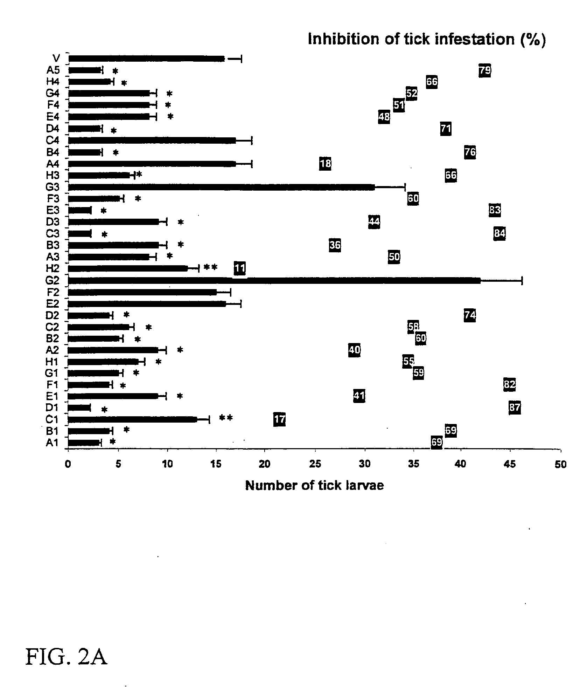

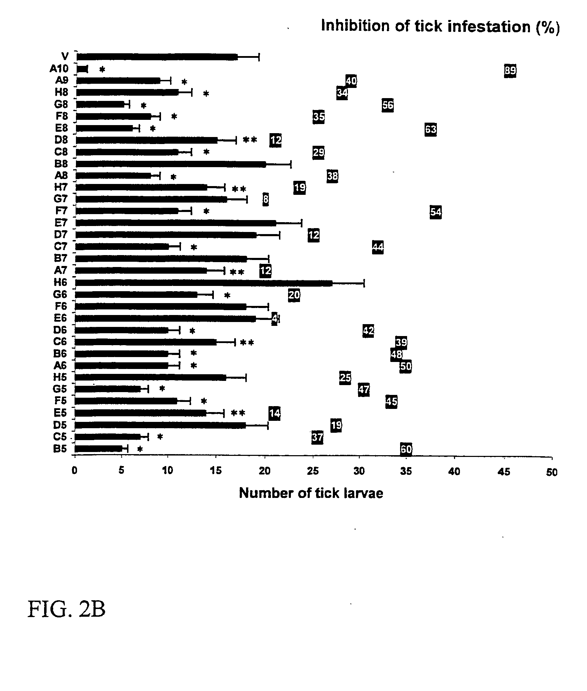

Pools of 41 (30-61) *I. scapularis* cDNA clones were screened by ELI. Only 33 cDNA pools and controls were analyzed per experiment. The average tick infestation level was 50±13 and 56±15 and 56±15 and 54±18 larvae/mouse for cDNA immunized and control mice, respectively (P>0.05) (Table 1). The average number of engorged larvae recovered per mouse was 9±3 and 13±4 in the cDNA-immunized mice and 16±4 and 17±3 in the control vector-immunized group (P<0.05) (Table 1). No reduction was observed in the number of larvae collected from mice that received the vector DNA compared to saline-immunized controls. The maximum number of engorged larvae was collected 3 to 4 days after infestation. However, in mice immunized with cDNA pools B5, A8 and A10 (Fig. 2) a retardation of larval development in 1 to 2 days was recorded. The average inhibition of tick infestation (I) was 49±28% and 30±22% (Table 1). After two experiments covering the analysis of 66 pools (2705 clones), 9 protective pools (351 clones) were selected producing an inhibition of tick infestation I≥60% (Fig. 2A and 2B and Table 1). When we started these experiments, we planed to screen over 4000 cDNA clones considering the complexity of the tick genome. However, to our surprise 9 protective cDNA pools were identified after screening 66 pools containing 2705 cDNA clones. This result probably reflects the possibility of interfering with tick infestations at many different levels that involve a Pleiades of gene products. Results from vaccination experiments against ticks employing recombinant antigens support this view (reviewed by Mulenga et al., 2000). Because of the complexity of the screening procedure in mice vaccinated and challenged with tick larvae, it was difficult to work with more than 9 protective cDNA pools. Therefore we did not continue screening new cDNA pools and focused our attention on the 9 pools selected after the primary screen.

### Secondary screen

The secondary screen was done to verify the protective capacity of the cDNA pools selected after the primary screen (Fig. 2A and 2B). After the primary screen of 66 cDNA pools (2705 clones), 9 pools with I≥60% were selected for the secondary screen (re-screening) employing 5 mice per group as described above. Engorged larvae were kept for molting in a 95% humidity atmosphere. Molting of engorged larvae was evaluated by visual examination of tick nymphs under a stereomicroscope 34 days after last larval collection. The inhibition of molting (M) for each test group was calculated with respect to vector-immunized controls as [1-(MLn/MLc x RLc/RLn)] x 100, where MLn is the number of nymphs for each test group, MLc is the number of nymphs for the control group, RLc is the number of larvae recovered for the control group, and RLi is the number of larvae recovered for each test group. Control mice were immunized with the negative (I=0%) F2 cDNA pool or saline only. A group was included immunized SC with two doses of 100 µg of total IDE8 tick cell proteins per dose in Freund's incomplete adjuvant.

All 9 protective cDNA pools gave positive results in the secondary screen (data not shown). The tick infestation levels were higher in this experiment (average 85±6 and 84±3 larvae/mouse for cDNA-immunized and control mice, respectively; P>0.05). Nevertheless, the average number of engorged larvae recovered per mouse was 39±7 and 26±6 for control and cDNA-immunized mice, respectively (P<0.05). The group immunized with total IDE8 tick cell proteins was protected with I=33%. Again, no reduction was observed in the number of larvae collected from mice that received the control cDNA (F2 negative pool after the primary screen; Fig. 2A) compared to saline-immunized controls.

In the secondary screen, molting of engorged larvae was evaluated after 34 days. Molting was affected in all but one test cDNA-immunized group. Inhibition of molting in test cDNA-immunized mice compared to the control cDNA-immunized group varied from 0% to 12% (6±4%). The inhibition of molting was higher than 50% only in the larvae collected from mice immunized with cDNA pools B5 and A10, which showed a retardation of larval development in 1 to 2 days as in the primary screen. No differences were observed between control cDNA and saline-immunized mice. Among the larvae that did not molt to nymph, some were visibly damaged and presented a strong red coloration. The percent of red larvae in cDNA-immunized mice varied between 3% to 18 % (7±5%) while in the saline and control cDNA-immunized groups red larvae represented the 6% and 4%, respectively.

### Tertiary screen

For the tertiary screen, 64 clones were grouped in 16 sub-pools each containing 1 to 17 plasmids according to the predicted function of encoded proteins (e.g., all the plasmids that encoded histone proteins were grouped together) and used with 4 sub-pools containing 182 clones of unknown function or with sequences without homology to sequence databases to immunize 4 mice per group. Mice were immunized with 0.3 µg/plasmid/dose in 50 µl PBS and evaluated as described above. Control mice were immunized with a pool of 20 plasmids containing mitochondrial cDNAs.

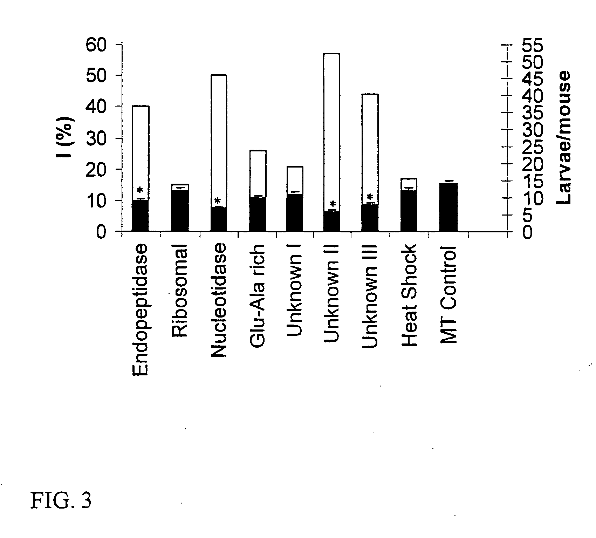

Tick infestation levels were similar in all test groups (72±2 larvae/mouse) and in control mice (69±2 larvae/mouse) (P>0.05). The number of engorged larvae recovered per mouse was also similar between test (16±7) and control (14±6) mice (P>0.05). However, the groups immunized with cDNA sub-pools containing clones with putative endopeptidase, nucleotidase, ribosomal proteins, heat shock proteins, glutamine-alanine-rich proteins and 3 of the sub-pools with unknown function or with sequences without homology to sequence databases had I≥15% (Fig. 3). Furthermore, among them, the groups immunized with sub-pools containing clones with a putative endopeptidase, nucleotidase and two of the cDNA sub-pools with unknown function or with sequences without homology to sequence databases resulted in lower infestation levels compared to control mice (P≤0.05) and I≥40% (Fig. 3). Clones homologous to chorion proteins, vitellogenin receptors, and peptidoglycan recognition proteins were selected for they potential protection capacity in other stages of tick development.

### Statistical analysis

The number of larvae attached per mouse and the number of engorged larvae recovered per mouse 7 days after infestation were compared by Analysis of Variance (ANOVA) followed by a series of Tukey's *post-hoc* tests for pair comparisons between cDNA-immunized and control vector DNA-immunized mice (primary screen), and by Student's t-test between mice immunized with positive cDNA pools and the control negative F2 cDNA pool (secondary screen) or between test cDNA sub-pools-immunized and control mice immunized with mitochondrial cDNAs (tertiary screen).

### Example 2: Sequence analysis of protective clones

All the 351 cDNA clones in the 9 pools that resulted positive in the secondary screen were sequenced. DNA from individual clones in these pools was purified (Wizard SV 96 plasmid DNA purification system, Promega) from the master plate and partially sequenced. In most cases a sequence larger than 700 nucleotides was obtained. Nucleotide sequences were analyzed using the program AlignX (Vector NTI Suite V 5.5, InforMax, North Bethesda, MD, USA). BLAST (Altschul et al., 1990) was used to search the NCBI databases to identify previously cloned sequences that may have homology to those that we sequenced. Sequence analysis allowed grouping the clones according to sequence identity to DNA databases and predicted protein function. The protective clones selected after the tertiary screen were fully sequenced.

Comparison to sequence databases permitted to identify sequence identity to previously reported genes with known function in 152 (43%) of the clones (Table 2). Fifty seven percent of the sequences were homologous to genes with unknown function or had no significant identity to previously reported sequences (Table 2). Of the clones with sequence identity to genes with known function, 85% were homologous to arthropod sequences. Ninety-three clones (61%) contained sequences homologous to *Drosophila melanogaster, 5* (3%) to other insects and 32 (21%) to Ixodid tick species. Thirty percent of the clones were eliminated from further analysis based on their sequence identity, including those containing similar sequences (Table 2). The protective clones included antigens homologous to endopeptidases, nucleotidases, chorion proteins, vitellogenin receptors, peptidoglycan recognition proteins, glutamine-alanine rich proteins, ribosomal proteins, and heat-shock proteins.

### Summary of results

The results obtained with the various protective clones identified in the Sequence Listing, along with certain selected expressed proteins, are summarized in Table 4.

SEQ ID NO: 1 denotes the clone designated 4E6, wherein the relevant protein encoding fragment has been identified as comprising residues 1-117, which encodes the polypeptide shown in SEQ ID NO: 2.

SEQ ID NO:3 denotes the clone designated 4D8, wherein the relevant protein encoding fragment has been identified as comprising residues 80-575, which encodes the polypeptide shown in SEQ ID NO: 4.

SEQ ID NO:5 denotes the clone designated 4F8, wherein the relevant protein encoding fragment has been identified as comprising residues 1-951, which encodes the polypeptide shown in SEQ ID NO: 6.

SEQ ID NO:7 denotes the clone designated 4G11, wherein the relevant protein encoding fragment has been identified as comprising residues 1-697, which encodes the polypeptide shown in SEQ ID NO: 8.

SEQ ID NO:9 denotes the clone designated 4D6, wherein the relevant protein encoding fragment has been identified as comprising residues 198-1025, which encodes the polypeptide shown in SEQ ID NO: 10.

SEQ ID NO:11 denotes the clone designated 3E1, wherein the relevant protein encoding fragment has been identified as comprising residues 3-578, which encodes the polypeptide shown in SEQ ID NO: 12.

SEQ ID NO:13 denotes the clone designated 1C10, wherein the relevant protein encoding fragment has been identified as comprising residues 1-1119, which encodes the polypeptide shown in SEQ ID NO: 14.

SEQ ID NO:15 denotes the clone designated 3E10, wherein the relevant protein encoding fragment has been identified as comprising residues 51-1544, which encodes the polypeptide shown in SEQ ID NO: 16.

SEQ ID NO:17 denotes the clone designated 4F11, wherein the relevant protein encoding fragment has been identified as comprising residues 31-2295, which encodes the polypeptide shown in SEQ ID NO: 18.

SEQ ID NO:19 denotes the clone designated 3C12, wherein the relevant protein encoding fragment has been identified as comprising residues 6-332, which encodes the polypeptide shown in SEQ ID NO: 20.

SEQ ID NO:21 denotes the clone designated 2C12, wherein the relevant protein encoding fragment has been identified as comprising residues 3-137, which encodes the polypeptide shown in SEQ ID NO: 22.

SEQ ID NOS: 22, 23 AND 24, denote, respectively, clones 1A9, 1B2 and 4A4, each comprising a partial sequence with no associated polypeptide.

It has now been discovered that certain of the protective antigens exhibit activity and are expressed across tick species, and in some circumstances across genera, thus making possible the use of these common antigens in broad range anti-tick vaccine formulations.

### Example 3: Extension of I. scapularis Protective Antigens To Other Tick Species

### Methods

### RNA extraction and reverse transcription (RT)-PCR

Total RNA was extracted from guts and salivary glands dissected from 30 unfed *I. scapularis* females, approximately 100 and 1000 *I. scapularis* nymphs and larvae, respectively, the egg mass oviposited by one *I. scapularis* female, salivary glands dissected from 10 *D. variabilis* males, salivary glands and guts dissected from 20 *A. americanum* and *R. sanguineus* adults, 10 *B. microplus* adults and 10 *I. pacificus* females, respectively and 100-150 *I. ricinus* larvae. Total RNA was extracted from homogenized tick samples using TRI Reagent (Sigma), except for *I. ricinus* and *B. microplus* RNA which were extracted using the RNA Instapur kit (Eurogentec, Seraing, Belgium) and the RNeasy mini kit (Qiagen, Valencia, CA, USA), respectively, according to the manufacturer's instructions. The final RNA pellet was resuspended in 50-100 µl diethyl pyrocarbonate-treated distilled deionized sterile water. RT-PCR reactions were performed using the Access RT-PCR system (Promega, Madison, WI, USA). One µl RNA was reverse transcribed in a 50 µl reaction mixture (1.5 mM MgSO₄, 1 X avian myeloblastosis virus (AMV) RT/*Thermus flavus* (*Tfl*) reaction buffer, 10 mM random hexamer, 0.2 mM each deoxynucleoside triphosphate (dNTP), 5 U AMV RT, 5u *Tfl* DNA polymerase (Promega), 10 pmol of each primer) at 48°C for 45 min. After 2 min incubation at 94°C, PCR was performed in the same reaction mixture with specific primers and amplification conditions (Table 5). Control reactions were performed using the same procedures but without RT to control for DNA contamination in the RNA preparations and without RNA added to control contamination of the PCR reaction. Positive control reactions for the PCR were performed with plasmid DNA containing the cloned tick cDNAs and with genomic DNA extracted from *I. scapularis* IDE8 cells. PCR products were electrophoresed on 1% agarose gels to check the size of amplified fragments by comparison to a DNA molecular weight marker (1 Kb Plus DNA Ladder, Promega).

### Cloning and sequencing of D. variabilis, A. americanum and B. microplus 4D8

Fragments of the *D. variabilis, A. americanum* and *B. microplus* 4D8 cDNA were amplified by RT-PCR and cloned into pGEM-T (Promega). Four clones of each were sequenced at the Core Sequencing Facility, Department of Biochemistry and Molecular Biology, Noble Research Center, Oklahoma State University.

### Vaccination of rabbits and challenge with I. scapularis, D. variabilis and A. americanum nymphs

An experiment was designed to evaluate the effect of tick protective antigens on nymphal stages of *I. scapularis, D. variabilis* and *A. americanum*. One New Zealand White rabbit per group was each immunized with 3 doses (weeks 0, 4 and 7) containing 50 µg/dose purified 4F8 and 4D8 proteins or 4E6 synthetic peptide derived from *I. scapularis,* a combination of 4F8+4D8+4E6 or bovine serum albumin (Sigma) as control in FIA (Sigma). Animals were cared for in accordance with standards specified in the Guide for Care and Use of Laboratory Animals. Rabbits were injected subcutaneously with 500 µl/dose using a 1 ml tuberculin syringe and a 27½G needle. Two weeks after the last immunization, rabbits were infested with 100 *I. scapularis* nymphs per rabbit in one ear and 110 nymphs of each *D. variabilis* and *A. americanum* on the other ear. The number of nymphs attached was recorded and engorged nymphs dropping from each rabbit's ear were collected daily and counted during 7 days. *D. variabilis* and *A. americanum* engorged nymphs were separated under a dissecting light microscope. Engorged nymphs collected after completion of the feeding period were counted and weighted. The inhibition of tick nymphal infestation was calculated with respect to the control group as described previously for larval tick infestations (Almazán et al., 2003). The reduction on the weight of engorged nymphs was determined in experimental groups with respect to the nymphs collected from the control group.

### Results

### Expression of protective cDNAs

Expression of genes encoding for tick protective antigens was analyzed at mRNA and protein levels by RT-PCR and immunohistochemistry, respectively. Expression of 4F8, 4D8 and 4E6 mRNA was detected in all *I. scapularis* developmental stages and in guts and salivary glands from adult ticks (Table 6). Control reactions ruled out contamination with genomic DNA or during the PCR. Furthermore, PCR of tick genomic DNA showed that the size of the amplified DNA fragments was higher than the size corresponding to cDNA fragments, probably due to the presence of intron sequences in the analyzed genes (Table 6).

Sequence conservation and expression of 4F8, 4D8 and 4E6 were analyzed in *I. scapularis* related species, *I. pacificus* and *I. ricinus*, and in *D. variabilis*, *R. sanguineus, B. microplus* and *A. americanum* by RT-PCR (Table 6) using the primers derived from *I. scapularis* sequences (Table 5). Expression was detected in *I. ricinus* for all three genes and in *I. pacificus* for 4D8 and 4E6. Expression of 4D8 was also detected in *D. variabilis, B. microplus, R. sanguineus* and *A. americanum.*

### Sequence of D. variabilis, A. americanum and B. microplus 4D8

The sequences of *D. variabilis, A. americanum and B. microplus* 4D8 cDNAs and their deduced amino acid sequences are provided in SEQ ID NOS: 26-31. The sequences associated with tick protective antigen 4D8 derived from Rhipicephalus appendiculatus are provide in SEQ ID NOS: 38-39.

### Protective properties of tick antigens against I. scapularis, D. variabilis and A. americanum nymphal infestations

The detection of 4D8 and 4E6 expression in *non-Ixodes* tick species were the basis for an experiment in which rabbits were immunized with *I. scapularis* derived protective antigens and challenged with *I. scapularis, D. variabilis* and *A. americanum* larvae. The results suggested an effect of 4D8 immunization on the inhibition of nymphal infestations in all three tick species and a notable effect on *D. variabilis* nymphs reflected in the number of visibly damaged ticks and the reduction in the weight of engorged nymphs (Table 7). Immunization with 4F8 had an effect on *I. scapularis* nymphal infestations only and the effect of 4E6 immunization was reflected on *A. americanum* infestations and on the weight of *D. variabilis* engorged nymphs (Table 7). The synergistic effect of the immunization with all three antigens was observed for 4D8 in *I. scapularis* nymphs (Table 7).

Experiment 3, then, demonstrates the surprising utility of using *I*. *scapularis* tick protective antigens 4D8, 4F8 and 4E6 in vaccine formulations for the control of multiple tick species.

Thus, the present invention relates to the identification of these antigens which are protective against a broad range of tick species, gene sequences and encoded proteins for such antigens, related vaccines and methods useful to induce an immune response. More generally, the invention concerns the given cDNA sequences and any nucleotide sequence coding for a protein which is capable of eliciting an antibody or other immune response (e.g., T-cell response of the immune system) which recognizes an epitope(s) of the amino acid sequences depicted in the Sequence Listing, including less than the full cDNA sequences and mutants thereof. Hence the nucleotide sequence may encode a protein which is the entire antigen encoded by the variously identified bases, or a fragment or derivative of the antigen or a fusion product of the antigen or fragment and another protein, provided that the protein which is produced from such sequence is capable of eliciting an antibody or other immune response which recognizes an epitope(s) of the given amino acid sequences.

As a result, the invention encompasses DNA sequences which encode for and/or express in appropriate transformed cells, proteins which may be the full length antigen, antigen fragment, antigen derivative or a fusion product of such antigen, antigen fragment or antigen derivative with another protein.

As will be appreciated by those of ordinary skill in the art, a nucleotide sequence encoding the inventive antigens or variants thereof may differ from the native sequences presented herein due to codon degeneracies, nucleotide polymorphisms, or nucleotide substitutions, deletions or insertions. While several embodiments of such molecules are depicted in the Sequence Listing, it should be understood that within the context of the present invention, reference to one or more of these molecules includes variants that are naturally occurring and/or synthetic sequences which are substantially similar to the sequences provided herein and, where appropriate, the protein (including peptides and polypeptides) that are encoded by these sequences and their variants. As used herein, the nucleotide sequence is deemed to be "substantially similar" if: (a) the nucleotide sequence is derived from the coding region of a native gene of the various noted tick species and encodes a peptide or polypeptide possessing the described antigenicity (including, for example, portions of the sequence or allelic variations of the provided sequences); or (b) the nucleotide sequences are degenerate (i.e., sequences which encode the same amino acid using a different codon sequence) as a result of the genetic code to the nucleotide sequences defined in (a); or (c) the nucleotide sequence is at least 95% identical to a nucleotide sequence provide herein, or (d) is a complement of any of the sequences described in (a-c).

Proteins included within the present invention have an amino acid sequence depicted in the Sequence Listing and homologs thereof. Other included proteins consist of a fragment of said sequence capable of eliciting an antibody or other immune response which recognizes an epitope(s) of the amino acid sequences depicted and a mutuant of said sequence capable of eliciting an antibody or other immune response which recognizes an epitope(s) of such amino acid sequences. With respect to homology to the native sequence, a variant should preferably have at least 95% amino acid sequence homology. As used herein, amino acid "homology" is determined by a computer algorithm incorporated in a protein database search program commonly used in the art, for example, as incorporated in the programs BLAST (BLAST.TM., a computer program) (Altschul et al., Nucleic Acids Res. (25) 3389-3402, 1997) or DNA STAR MEGALIGN (DNA STAR MEGALIGN.TM., a computer program) which return similar results in homology calculations.

The nucleotide sequences may be inserted into any of a wide variety of expression vectors by a variety of procedures. Such procedures and others are deemed to be known by those skilled in the art. Suitable vectors include chromosomal, nonchromosomal and synthetic DNA sequences; e.g., derivatives of SV40; bacterial plasmids; phage DNAs; yeast plasmids; vectors derived from combinations of plasmids and phage DNAs, viral DNA such as baculovirus, vaccinia, adenovirus, fowl pox virus, pseudorabies, etc. The appropriate DNA sequence must be operatively linked in the vector to an appropriate expression control sequence(s) (promoter) to direct mRNA synthesis. As representative examples of such promoters, there may be mentioned LTR or SV40 promoter, the E. coli lac or trp, the phage lambda PL promoter and other promoters known to control expression of genes in prokaryotic and eukaryotic cells or their viruses. The expression vector also includes a non-coding sequence for a ribosome binding site for translation initiation and a transcription terminator. The vector may also include appropriate sequences for amplifying expression.

The vector containing the appropriate cDNA sequence as hereinabove described, as well as an appropriate promoter or control sequence, may be employed to transform an appropriate host to permit the host to express the protein. Examples of host organisms and cells include bacterial strains (e.g., *E. coli*, Pseudomonas, *Bacillus, Salmonella,* etc.), fungi (e.g., yeasts and other fungi), animal or plant hosts (e.g., mouse, swine or animal and human tissue cells). The selection of the host is deemed to be within the scope of those skilled in the art.

It is also understood that the appropriate cDNA sequence present in the vector when introduced into a host may express part or only a portion of the protein which is encoded within the noted terminology, it being sufficient that the expressed protein be capable of eliciting an antibody or other immune response which recognizes an epitope(s) of the listed amino acid sequences.

The isolated cDNAs and/or polypeptide expressed by the host transformed by the vector may be harvested by methods which will occur to those skilled in the art and used in a vaccine for protection of a mammal, such as dogs, cattle, humans, etc., against infestations of *Ixodes* and *non-Ixodes* tick species. Such protective recombinant proteins and/or modified cDNAs are used in an amount effective to induce an immune response against such ticks and their associated pathogens and may be used in combination with a suitable physiologically acceptable carrier. The term "inducing an immune response" when used with respect to the vaccine described herein means that the vaccine prevents disease associated with a particular tick species or reduces the severity of the disease.

The carrier employed in conjunction with vaccine may be any one of a wide variety of carriers. As representative examples of suitable carriers, there may be mentioned mineral oil, synthetic polymers, etc. Carriers for vaccines are well known in the art and the selection of a suitable carrier is deemed to be within the scope of those skilled in the art. The selection of a suitable carrier is also dependent upon the manner in which the vaccine is to be administered.

The present invention provides a method of immunizing a susceptible mammal, against infestations and disease caused by ticks with the vaccine described above. For purposes of this invention, the vaccine is administered in an effective amount. The vaccine may be administered by any of the methods well known to those skilled in the art, for example, by intramuscular, subcutaneous, intraperitoneal or intravenous injection. Alternatively, the vaccine may be administered intranasally or orally. It is also to be understood that the vaccine may include active components, such as tick-borne pathogen components or adjuvants in addition to the antigen(s) or fragments hereinabove described.

The host expressing the antigen may itself be used to deliver antigen to non-human animals, by introducing killed or viable host cells that are capable of propagating in the animal. Direct incorporation of the cDNA sequences into host cells may also be used to introduce the sequences into animal cells for expression of antigen in vivo.

### Bibliography

The following references are incorporated herein by reference:
Alberti E, Acosta A, Sarmiento ME, Hidalgo C, Vidal T, Fachado A, Fonte L, Izquierdo L, Infante JF, Finlay CM, Sierra G. Specific cellular and humoral immune response in Balb/c mice immunised with an expression genomic library of *Trypanosoma cruzi.* Vaccine 1998; 16: 608-12.
Almazán C, Kocan KM, Bergman DK, Garcia-Garcia JC, Blouin EF, de la Fuente J. Identification of protective antigens for the control of *Ixodes scapularis* infestations using cDNA expression library immunization. Vaccine 21, 1492-1501 (2003).
Almazán C, Kocan KM, Bergman DK, Garcia-Garcia JC, Blouin EF, de la Fuente J. Characterization of genes transcribed in an *Ixodes scapularis* cell line that were identified by expression library immunization and analysis of expressed sequence tags. *Gene Therapy and Molecular Biology,* 7:9-25 (2000).
Altschul SF, Gish W, Miller W, Myers EW, Lipman DJ. Basic local alignment search tool. J Mol Biol 1990; 215: 403-10.
Barry MA, Lai WC, Johnston SA. Protection against mycoplasma infection using expression-library immunization. Nature 1995; 377: 632-5.
Black WC 4th, Piesman J. Phylogeny of hard- and soft-tick taxa (Acari: Ixodida) based on mitochondrial 16S rDNA sequences. Proc Natl Acad Sci U S A 1994; 91: 10034-8.
Brayton KA, Vogel SW, Allsopp BA. Expression library immunization to identify protective antigens from *Cowdria ruminantium.* Ann N Y Acad Sci 1998; 849: 369-71.
Cassataro J, Velikovsky CA, Giambartolomei GH, Estein S, Bruno L, Cloeckaert A, Bowden RA, Spitz M, Fossati CA. Immunogenicity of the *Brucella melitensis* recombinant ribosome recycling factor-homologous protein and its cDNA. Vaccine 2002; 20: 1660-9.
de la Fuente J, Kocan KM. 2003. Advances in the identification and characterization of protective antigens for development of recombinant vaccines against tick infestations. *Expert Review of Vaccines*. 2:583-593.
de la Fuente J, Rodriguez M, Redondo M, Montero C, Garcia-Garcia JC, Mendez L, Serrano E, Valdes M, Enriquez A, Canales M, Ramos E, Boue O, Machado H, Lleonart R, de Armas CA, Rey S, Rodriguez JL, Artiles M, Garcia L. Field studies and cost-effectiveness analysis of vaccination with Gavac against the cattle tick *Boophilus microplus.* Vaccine 1998; 16: 366-73.
de la Fuente J, Rodriguez M, Montero C, Redondo M, Garcia-Garcia JC, Mendez L, Serrano E, Valdes M, Enriquez A, Canales M, Ramos E, Boue O, Machado H, Lleonart R. Vaccination against ticks (*Boophilus* spp.): the experience with the Bm86-based vaccine Gavac. Genet Anal 1999; 15: 143-8.
de la Fuente J, Rodriguez M, Garcia-Garcia JC. Immunological control of ticks through vaccination with *Boophilus microplus* gut antigens. Ann N Y Acad Sci 2000; 916: 617-21.
De Rose R, McKenna RV, Cobon G, Tennent J, Zakrzewski H, Gale K, Wood PR, Scheerlinck JP, Willadsen P. Bm86 antigen induces a protective immune response against *Boophilus microplus* following DNA and protein vaccination in sheep. Vet Immunol Immunopathol 1999; 71: 151-60.
de Vos S, Zeinstra L, Taoufik O, Willadsen P, Jongejan F. Evidence for the utility of the Bm86 antigen from *Boophilus microplus* in vaccination against other tick species. Exp Appl Acarol 2001; 25: 245-61.
Drew DR, Lightowlers M, Strugnell RA. Vaccination with plasmid DNA expressing antigen from genomic or cDNA gene forms induces equivalent humoral immune responses. Vaccine 1999; 18: 692-702.
Elad D, Segal E. Immunogenicity in calves of a crude ribosomal fraction of *Trichophyton verrucosum:* a field trial. Vaccine 1995; 13: 83-7.
Estrada-Peña A, Jongejan F. Ticks feeding on humans: a review of records on human-biting Ixodoidea with special reference to pathogen transmission. Exp Appl Acarol 1999; 23: 685-715.
Garcia-Garcia JC, Gonzalez IL, Gonzalez DM, Valdes M, Mendez L, Lamberti J, D'Agostino B, Citroni D, Fragoso H, Ortiz M, Rodriguez M, de la Fuente J. Sequence variations in the *Boophilus microplus* Bm86 locus and implications for immunoprotection in cattle vaccinated with this antigen. Exp Appl Acarol 1999; 23: 883-95.
Kofta W, Wedrychowicz H. c-DNA vaccination against parasitic infections: advantages and disadvantages. Vet Parasitol 2001; 100: 3-12.
Liyou N, Hamilton S, Elvin C, Willadsen P. Cloning and expression of ecto 5'-nucleotidase from the cattle tick *Boophilus microplus.* Insect Mol Biol 1999; 8: 257-66.
Liyou N, Hamilton S, Mckenna R, Elvin C, Willadsen P. Localization and functional studies on the 5'-nucleotidase of the cattle tick *Boophilus microplus.* Exp Appl Acarol 2000; 24: 235-46.
Manoutcharian K, Terrazas LI, Gevorkian G, Govezensky T. Protection against murine cysticercosis using cDNA expression library immunization. Immunol Lett 1998; 62: 131-6.
Melby PC, Ogden GB, Flores HA, Zhao W, Geldmacher C, Biediger NM, Ahuja SK, Uranga J, Melendez M. Identification of vaccine candidates for experimental visceral leishmaniasis by immunization with sequential fractions of a cDNA expression library. Infect Immun 2000; 68: 5595-602.
Moore RJ, Lenghaus C, Sheedy SA, Doran TJ. Improved vectors for expression library immunization--application to *Mycoplasma hyopneumoniae* infection in pigs. Vaccine 2001; 20: 115-20.
Mulenga A, Sugimoto C, Onuma M. Issues in tick vaccine development: identification and characterization of potential candidate vaccine antigens. Microbes Infect 2000; 2: 1353-61.
Munderloh UG, Wang YLM, Chen C, Kurtti TJ. Establishment, maintenance and description of cell lines from the tick *Ixodes scapularis.* J Parasitol 1994; 80: 533-43.
Nuttall PA. Pathogen-tick-host interactions: *Borrelia burgdorferi* and TBE virus. Zentralbl Bakteriol 1999; 289: 492-505.
Parola P, Raoult D. Tick-borne bacterial diseases emerging in Europe. Clin Microbiol Infect 2001; 7: 80-3.
Silva CL. The potential use of heat-shock proteins to vaccinate against mycobacterial infections. Microbes and Infection 1999; 1: 429-35.
Singh RA, Wu L, Barry MA. Generation of genome-wide CD8 T cell responses in HLA-A*0201 transgenic mice by an HIV-1 ubiquitin expression library immunization vaccine. J Immunol 2002; 168: 379-91.
Smooker PM, Setiady YY, Rainczuk A, Spithill TW. Expression library immunization protects mice against a challenge with virulent rodent malaria. Vaccine 2000; 18: 2533-40.
van Drunen Littel-van den Hurk S, Loehr BI, Babiuk LA. Immunization of livestock with DNA vaccines: current studies and future prospects. Vaccine 2001; 19: 2474-9.
Wikel SK, Ramachandra RN, Bergman DK, Burkot TR, Piesman J. Infestation with pathogen-free nymphs of the tick *Ixodes scapularis* induces host resistance to transmission of *Borrelia burgdorferi* by ticks. Infect Immun 1997; 65: 335-8.Willadsen P. Novel vaccines for ectoparasites. Vet Parasitol 1997; 71: 209-22.
Willadsen P, Jongejan F. Immunology of the tick-host interaction and the control of ticks and tick-borne diseases. Parasitol Today 1999; 15: 258-62.

**Table 1. Primary screen of the I. scapularis cDNA library by ELI in mice.**

| Experimental group^{a} | Number of pools screened (Number of clones) | Average±SD number of larvae attached per mouse^{b} | Average±SD number of engorged larvae per mouse^{c} | Average±SD inhibition of tick infestation (I)^{d} | Number of pools selected for the secondary screen |
|---|---|---|---|---|---|
| Experiment 1 | 33 (1383) | 50±13 (33-80) | 9±3 (2-42) | 39±55% (-183 - 87%) | 6 (I>75%) |
| Vector DNA-immunized controls for experiment 1 | - - - | 56±13 (45-67) | 16±4 (5-27) | - - - | - - - |
| Experiment 2 | 33 (1322) | 56±15 (29-79) | 13±4 (1-27) | 27±28% (-53 - 89%) | 3 (I>60%) |
| Vector DNA-immunized controls for experiment 2 | - - - | 54±18 (36-73) | 17±3 (6-28) | - - - | - - - |

| | | | | | |
|---|---|---|---|---|---|
| ^{a}Ninety six LBA plates containing an average of 41 cDNA clones per plate were prepared. Replicas were made and clones from each plate were pooled, inoculated, grown for 2 hr in a 96 wells plate and plasmid DNA purified from each pool for ELI. Three mice per group were each immunized IM twice with 1 µg DNA/dose in 50 µl PBS two weeks apart. Two groups of 3 mice each were included as controls. One group was injected with vector DNA and the second with saline only. | | | | | |
| ^{b}Fifteen days after the last immunization, mice were infested with 100 *I. scapularis* larvae per mouse. Twelve hrs later, larvae that did not attach were counted to calculate the number of attached larvae per mouse and mice were transferred to new cages. | | | | | |
| ^{c}Engorged larvae dropping from each mouse were collected daily and counted after 7 days. | | | | | |
| ^{d}The inhibition of tick infestation (I) for each test group was calculated with respect to vector-immunized controls as [1-(RLn/RLc x RLic/RLin)] x 100, where RLn is the average number of replete larvae recovered per mouse for each test group, RLc is the average number of replete larvae recovered per mouse for control group, RLic is the average number of larvae attached per mouse for control group, and RLin is the average number of larvae attached per mouse for each test group. | | | | | |

**Table 2. Classification of the clones in protective pools by putative protein function according to identity to sequence databases.**

| **Putative protein Function** | **Number of clones** |
|---|---|
| Biosynthetic^{a} | 2 |
| Catabolism | 4 |
| Cell adhesion | 2 |
| Cell cycle^{a} | 2 |
| Cytoskeletal^{a} | 8 |
| Defense | 2 |
| DNA structure or replication^{a} | 3 |
| Extracellular matrix | 3 |
| Endocytosis | 2 |
| Energy metabolism | 10 |
| Homeostasis | 2 |
| Morphogenetic | 9 |
| Mitochondrial^{a} | 34 |
| Protein synthesis or processing^{a,b} | 34 |
| RNA synthesis or processing^{a} | 7 |
| Heat-shock proteins | 4 |
| Signal transduction | 16 |
| Transport | 8 |
| Unknown | 199 |
| Total | 351 |

| | |
|---|---|
| ^{a}Eliminated from further screening of protective antigens. Other clones were eliminated for containing similar sequences. | |
| ^{b}Except for ribosomal proteins. | |

**Table 3. Grouping of the clones according to the predicted function of encoded proteins in sub-pools for the tertiary screen.**

| **Sub-pool (No. of clones)** | **Clone** | **Pool**^{**a**} |
|---|---|---|
| Ribosomal (17) | 1A2, 1A10, 1C11 1F6 2B8 2F8, 2F10 3A10, 2C3, 3D2, 3D10 3G9, 3G10 4D11, 4D12, 4E7, 4F7 | A5 D1 A10 E8 B4 E3 F1 |
| Membrane protein (7) | 1D8, 1D11, 1E10 2B12 2H5 3C9 3G11 | D1 A10 E8 B4 E3 |
| ATPase (6) | 1A9, 1B2, 1C9 2C9 4A4 4G12 | A5 A10 C3 F1 |
| Cell channel/Transporter (5) | 1F4 2H11 4A12 4G10, 4G11 | D1 E8 C3 F1 |
| Early development-specific (4) | 1C8 3F4 4C7 4G9 | A5 E3 C3 F1 |
| G protein-coupled receptor (4) | 2B7, 2C12 2F12 4C9 | A10 E8 C3 |
| Growth factor receptor (3) | 2E8 3B8, 3C8 | B5 B4 |
| Lectin (3) | 3E10 4B8,4C8 | E3 C3 |
| Vitellogenin (3) | 1F12 4A6 4G2 | D1 C3 F1 |
| Heat shock (3) | 1C10 1F10 3F6 | A5 D1 E3 |
| EGF-like (2) | 2H4 4C10 | E8 C3 |
| Secreted protein (2) | 2F9 3C12 | E8 B4 |
| Glutamine-Alanine rich (2) | 4D6, 4E6 | F1 |
| Adaptin (1) | 3E1 | E3 |
| Endopeptidase (1) | 4D8 | F1 |
| Nucleotidase (1) | 4F8 | F1 |

| | | |
|---|---|---|
| ^{a}cDNA pools refer to positive pools after primary and secondary screens (Fig. 2A and 2B). | | |

**Table 4: Summary of results with I. scapularis cDNA clones.**

| **cDNA clone** | **Predicted Protein** | **Inhibition of tick infestation I (%)** | **Inhibition of molting M (%)** | **Efficacy E (%)** |
|---|---|---|---|---|
| 4D8 | Endopeptidase | 40*/54** | 7*/8** | 44*/58** |
| 4F8 | Nucleotidase | 50*/64** | 17*/-9** | 58*/61** |
| 1C10 | HSP70 | 17* | ND | ND |
| 4D6 | Glu-Ala-rich | 61 * | 11 | 66* |
| 4E6 | Glu-Ala-rich | 20*/46** | 16** | 55** |
| 3E1 | β-adaptin (appendage region) | 27* | 5* | 31* |
| 2C12 | Beta-amyloid precursor protein (APP) | -8*** | ND | ND |
| 4F11 | Block of proliferation Bop1 | -39*** | ND | ND |
| 3E10 | Mannose binding lectin | -48*/-10*** | ND | ND |
| 4G11 | Chloride channel | 38*** | 30 | 57 |
| 3C12 | RNA polymerase III | -104*** | ND | ND |
| 1A9, 1B2, 4A4 | ATPase | -57*** | ND | ND |

| | | | | |
|---|---|---|---|---|
| Mice were immunized with cDNA-containing expression plasmid DNA as described above (*) or with 100 µg/dose of recombinant protein expressed in *E. coli* (**). I, M and E were calculated as described above. ND, not determined. | | | | |
| ***Resulted in a pro-feeding activity. This effect could be due to the expression of cDNAs encoding for tick immunosuppressants, anticoagulants and other proteins with low antigenicity and a pro-feeding activity. Alternatively, they could encode for proteins homologous to host proteins with anti-tick activity, which neutralization results in a tick pro-feeding activity. | | | | |

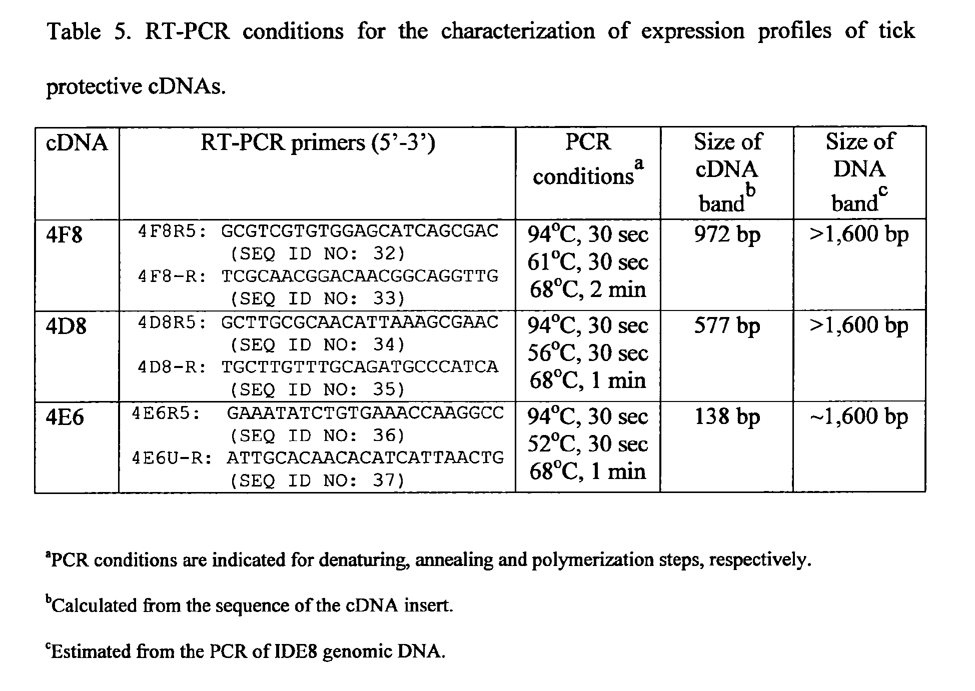

**Table 6. Expression profile of mRNAs encoding for protective antigens in ticks.**

| | *I. scapularis* | | | | | | *I. pacificus* | *I. ricinus* | *A. americanum* | *D. variabilis* | *R. sanguineus* | *B. microplus* |
|---|---|---|---|---|---|---|---|---|---|---|---|---|
| RNA source | I D E 8 | e g g s | l a r v a e | n y m p h s | g u t s | salivary g l a n d s | | | | | | |
| Clone | mRNA expression | | | | | | | | | | | |
| 4F8 | + | + | + | + | + | + | - | + | - | - | - | - |
| 4D8 | + | + | + | + | + | + | + | + | + | + | + | + |
| 4E6 | + | + | + | + | + | + | + | + | + | - | - | - |

| | | | | | | | | | | | | |
|---|---|---|---|---|---|---|---|---|---|---|---|---|
| Expression of mRNAs encoding for tick protective antigens 4F8, 4D8 and 4E6 was analyzed by RT-PCR in different tick species using the primers derived from *I. scapularis* sequences. | | | | | | | | | | | | |

**Table 7. Results of vaccination with recombinant tick protective antigens on I. scapularis, D. variabilis and A. americanum nymphs.**

| Tick species | Inhibition of tick nymphal infestation (%)^{a} | | | | Reduction in the weight of engorged nymphs (%)^{b} | | | |
|---|---|---|---|---|---|---|---|---|
| | 4D8 | 4F8 | 4E6 | All | 4D8 | 4F8 | 4E6 | All |
| *I. scapularis* | 35 | 39 | 0 | 63 | 0 | 0 | 0 | 0 |
| *D. variabilis* | 22 | 0 | 5 | 8 | 32 | 0 | 27 | 0 |
| *A. americanum* | 17 | 9 | 36 | 12 | 3 | 1 | 0 | 16 |

| | | | | | | | | |
|---|---|---|---|---|---|---|---|---|
| ^{a}Determined with respect to the number of engorged nymphs recovered from the control group as described above for tick larval infestations. | | | | | | | | |
| ^{b}Average weight per engorged nymph with respect to the weight of nymphs collected from the control group. | | | | | | | | |

In view of the above, it will be seen that the several objectives of the invention are achieved and other advantageous results attained. As various changes could be made in the above DNA molecules, proteins, etc. without departing from the scope of the invention, it is intended that all matter contained in the above description or shown in the accompanying drawings shall be interpreted as illustrative and not in a limiting sense. While the invention has been described with a certain degree of particularity, it is understood that the invention is not limited to the embodiment(s) set for herein for purposes of exemplification, but is to be limited only by the scope of the attached claim or claims, including the full range of equivalency to which each element thereof is entitled.

## Claims

1. An isolated polypeptide selected from the group consisting of tick derived polypeptides designated 4D8, 4F8 and 4E6.

2. A multi species anti-tick vaccine composition comprising the polypeptide of claim 1 and a pharmaceutically acceptable carrier or diluent.

3. The isolated polypeptide of claim 1, wherein the polypeptide comprises the tick derived polypeptide 4D8.

4. An isolated polypeptide comprising the amino acid sequence of at least one of SEQ ID NO:4, SEQ ID NO:27, SEQ ID NO:29, SEQ ID NO: 31, or SEQ ID NO:39.

5. A multi species anti-tick vaccine composition comprising the polypeptide of claim 4 and a pharmaceutically acceptable carrier or diluent.

6. The polypeptide of claim 4, wherein the polypeptide comprises the amino acid sequence of SEQ ID NO:4.

7. The polypeptide of claim 4, wherein the polypeptide comprises the amino acid sequence of SEQ ID NO:27.

8. The polypeptide of claim 4, wherein the polypeptide comprises the amino acid sequence of SEQ ID NO:29.

9. The polypeptide of claim 4, wherein the polypeptide comprises the amino acid sequence of SEQ ID NO:31.

10. The polypeptide of claim 4, wherein the polypeptide comprises the amino acid sequence of SEQ ID NO:39.

11. An isolated polypeptide comprising an amino acid sequence that is at least 95% homologous to at least one of SEQ ID NO:4, SEQ ID NO:27, SEQ ID NO:29, SEQ ID NO: 31, or SEQ ID NO:39.

12. An isolated cDNA molecule which encodes a tick derived antigenic polypeptide, said molecule having a nucleotide sequence that is at least 95% homologous to at least residues 1-117 of SEQ ID NO: 1.

13. An expression vector comprising the isolated cDNA molecule of claim 12.

14. An isolated cell transformed by the expression vector of claim 13.

15. A multi species anti-tick vaccine comprising an effective immunizing amount of an immunogen selected from the group consisting of (i) the isolated cDNA molecule of claim 12, (ii) the polypeptide encoded by the isolated cDNA molecule of claim 12, and (iii) a combination of the isolated cDNA molecule of claim 12 and the encoded polypeptide, and further comprising a pharmaceutically acceptable carrier or diluent.

16. A method of inducing an immune response in a mammal against multi species tick infestation and their associated pathogens, comprising administering to the mammal the vaccine of claim 15.

17. The polypeptide encoded by the isolated cDNA molecule of claim 12.

18. An isolated cDNA molecule which encodes a tick derived antigenic polypeptide, said molecule having a nucleotide sequence that is at least 95% homologous to at least residues 80-575 of SEQ ID NO: 3.

19. An expression vector comprising the isolated cDNA molecule of claim 18.

20. An isolated cell transformed by the expression vector of claim 19.

21. A multi species anti-tick vaccine comprising an effective immunizing amount of an immunogen selected from the group consisting of (i) the isolated cDNA molecule of claim 18, (ii) the polypeptide encoded by the isolated cDNA molecule of claim 18, and (iii) a combination of the isolated cDNA molecule of claim 18 and the encoded polypeptide, and further comprising a pharmaceutically acceptable carrier or diluent.

22. A method of inducing an immune response in a mammal against multi species tick infestation and their associated pathogens, comprising administering to the mammal the vaccine of claim 21.

23. The polypeptide encoded by the isolated cDNA molecule of claim 18.

24. An isolated cDNA molecule which encodes a tick derived antigenic polypeptide, said molecule having a nucleotide sequence that is at least 95% homologous to at least residues 10-951 of SEQ ID NO: 5.

25. An expression vector comprising the isolated cDNA molecule of claim 24.

26. An isolated cell transformed by the expression vector of claim 25.

27. A multi species anti-tick vaccine comprising an effective immunizing amount of an immunogen selected from the group consisting of (i) the isolated cDNA molecule of claim 24, (ii) the polypeptide encoded by the isolated cDNA molecule of claim 24, and (iii) a combination of the isolated cDNA molecule of claim 24 and the encoded polypeptide, and further comprising a pharmaceutically acceptable carrier or diluent.

28. A method of inducing an immune response in a mammal against multi species tick infestation and their associated pathogens, comprising administering to the mammal the vaccine of claim 27.

29. The polypeptide encoded by the isolated cDNA molecule of claim 24.

30. An isolated cDNA molecule which encodes a tick derived antigenic polypeptide, said molecule having a nucleotide sequence that is at least 95% homologous to SEQ ID NO: 26.

31. An expression vector comprising the isolated cDNA molecule of claim 30.

32. An isolated cell transformed by the expression vector of claim 31.

33. A multi species anti-tick vaccine comprising an effective immunizing amount of an immunogen selected from the group consisting of (i) the isolated cDNA molecule of claim 30, (ii) the polypeptide encoded by the isolated cDNA molecule of claim 30, and (iii) a combination of the isolated cDNA molecule of claim 30 and the encoded polypeptide, and further comprising a pharmaceutically acceptable carrier or diluent.

34. A method of inducing an immune response in a mammal against multi species tick infestation and their associated pathogens, comprising administering to the mammal the vaccine of claim 33.

35. The polypeptide encoded by the isolated cDNA molecule of claim 30.

36. An isolated cDNA molecule which encodes a tick derived antigenic polypeptide, said molecule having a nucleotide sequence that is at least 95% homologous to SEQ ID NO: 28.

37. An expression vector comprising the isolated cDNA molecule of claim 36.

38. An isolated cell transformed by the expression vector of claim 37.

39. A multi species anti-tick vaccine comprising an effective immunizing amount of an immunogen selected from the group consisting of (i) the isolated cDNA molecule of claim 36, (ii) the polypeptide encoded by the isolated cDNA molecule of claim 36, and (iii) a combination of the isolated cDNA molecule of claim 36 and the encoded polypeptide, and further comprising a pharmaceutically acceptable carrier or diluent.

40. A method of inducing an immune response in a mammal against multi species tick infestation and their associated pathogens, comprising administering to the mammal the vaccine of claim 39.

41. The polypeptide encoded by the isolated cDNA molecule of claim 36.

42. An isolated cDNA molecule which encodes a tick derived antigenic polypeptide, said molecule having a nucleotide sequence that is at least 95% homologous to SEQ ID NO: 30.

43. An expression vector comprising the isolated cDNA molecule of claim 42.

44. An isolated cell transformed by the expression vector of claim 43.

45. A multi species anti-tick vaccine comprising an effective immunizing amount of an immunogen selected from the group consisting of (i) the isolated cDNA molecule of claim 42, (ii) the polypeptide encoded by the isolated cDNA molecule of claim 42, and (iii) a combination of the isolated cDNA molecule of claim 42 and the encoded polypeptide, and further comprising a pharmaceutically acceptable carrier or diluent.

46. A method of inducing an immune response in a mammal against multi species tick infestation and their associated pathogens, comprising administering to the mammal the vaccine of claim 45.

47. The polypeptide encoded by the isolated cDNA molecule of claim 42.

48. An isolated cDNA molecule which encodes a tick derived antigenic polypeptide, said molecule having a nucleotide sequence that is at least 95% homologous to SEQ ID NO: 38.

49. An expression vector comprising the isolated cDNA molecule of claim 48.

50. An isolated cell transformed by the expression vector of claim 49.

51. A multi species anti-tick vaccine comprising an effective immunizing amount of an immunogen selected from the group consisting of (i) the isolated cDNA molecule of claim 48, (ii) the polypeptide encoded by the isolated cDNA molecule of claim 48, and (iii) a combination of the isolated cDNA molecule of claim 48 and the encoded polypeptide, and further comprising a pharmaceutically acceptable carrier or diluent.

52. A method of inducing an immune response in a mammal against multi species tick infestation and their associated pathogens, comprising administering to the mammal the vaccine of claim 51.

53. The polypeptide encoded by the isolated cDNA molecule of claim 48.
